# EUROPÄISCHE PATENTSCHRIFT

(11) **EP 2 359 872 B1**
(45) Veröffentlichungstag und Bekanntmachung des Hinweises auf die Patenterteilung: **20.08.2014**
(21) Anmeldenummer: 10015754.4
(22) Anmeldetag: 17.12.2010
(51) Int. Cl.: A61L 24/00, A61L 27/44, A61L 24/06

(54) **Dreikomponentenknochenzement**
Triple component bone cement
Ciment osseux à trois composants

(30) Priorität: 27.01.2010 DE 102010005956
(43) Veröffentlichungstag der Anmeldung: 24.08.2011
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 90491 Nürnberg (DE)
(74) Vertreter: Kühn, Hans-Christian

(56) Entgegenhaltungen:
- EP-A2- 2 052 747
- DE-B3-102007 050 762

## Beschreibung

Die vorliegende Erfindung betrifft einen Kit zur Herstellung von Knochenzement, eine Paste zur Herstellung von Knochenzement und Verwendungen des Kits und der Paste.

Knochenzemente auf der Basis von Polymethylmethacrylat (PMMA) sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Gleichzeitig reagiert der Aktivator N,N-dimethyl-p-toluidin mit dem Dibenzoylperoxid, dass unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs, bis der Zementteig erstarrt und damit ausgehärtet ist.

Die grundlegenden mechanischen Anforderungen an PMMA-Knochenzemente, wie 4-Punkt-Biegefestigkeit, Biegemodul und Druckfestigkeit, sind in der ISO 5833 beschrieben. Für den Anwender der PMMA-Knochenzemente ist die Eigenschaft der Klebfreiheit des Knochenzementes von wesentlicher Bedeutung. Der Begriff Klebfreiheit ist in der ISO 5833 definiert. Die Klebfreiheit zeigt bei konventionellen PMMA-Knochenzementen an, dass der Zement nach dem Vermischen der Komponenten durch Quellung der im Zementpulver enthaltenen Polymere im Monomer die Verarbeitungsphase erreicht hat. Grundsätzlich muss ein PMMA-Knochenzement klebfrei sein, damit der Anwender den Zement formen und applizieren kann. Der PMMA-Knochenzement darf nicht an den Handschuhen und an Applikationshilfen, wie Mischsystemen, Tiegeln oder Spateln kleben.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen können. Das Vermischen der Komponenten muss zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verweridung von Vakuummischsystemen wird im Gegensatz zur Handanmischung die Bildung von Luftblasen im Zementteig weitgehend verhindert, jedoch ist für diese Systeme eine zusätzliche Vakuumpumpe erforderlich. Beispiele für Mischsysteme sind in den Schriften US 4,015,945 A, EP 0 674 888 A1 und JP 2003181270 A offengelegt. Vakuummischsysteme und Vakuumpumpen sind relativ kostenintensiv. Nach der Vermischung der Monomerkomponente mit der Pulverkomponente muss je nach Zementtyp eine mehr oder weniger lange Zeit gewartet werden, bis der Zementteig klebfrei ist und appliziert werden kann. Aufgrund der vielen Fehlermöglichkeiten beim Anmischen von konventionellen PMMA-Knochenzementen wird entsprechend geschultes Personal benötigt. Die Schalungen sind mit einem nicht geringen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Um die bei der Herstellung eines Knochenzementes aus einer flüssigen Monomerlösung und einem Polymerpulver zu verhindern, schlägt die DE 102007050762 B3 einen pastösen Knochenzement vor. Dieser pastöse Knochenzement basiert auf der Idee, ein Polymer in einem Methacrylatmonomer zu lösen und darin ein partikuläres, nicht im Methacrylatmonomer lösliches Polymer zu suspendieren. Dadurch gelingt es, eine teigartige Masse herzustellen, die durch das gelöste Polymer einen hohen inneren Zusammenhalt zeigt und die durch das partikuläre, unlösliche Polymer eine so hohe Viskosität hat, dass der Teig kurzzeitig dem Blutungsdruck standhalten kann. Durch radikalische Polymerisation des Methacrylatmonomeren kann der Teig ausgehärtet werden. Die radikalische Polymerisation gelingt mit (i) einem radikalischen Initiator, wie Barbitursäurederivaten oder Dibenzoylperoxid, und (ii) einem Kupfersalz als Aktivator. Es hat sich jedoch gezeigt, dass bei Verwendung dieses Initiatorsystems der geformte Knochenzementteig nicht gleichmäßig, sondern vom Kern nach außen in Richtung Oberfläche des geformten Knochenzementteigs aushärtet. Durch Verdampfung des im Knochenzementteig enthaltenen Monomers kommt es dabei zur Bildung von Blasen im entstehenden Knochenzement. Ferner wurde beobachtet, dass bei Verwendung dieses Initiatorsystems die im Knochenzementteig enthaltenen Monomere nicht vollständig umgesetzt werden. Diese Umstände wirken sich nachteilig auf die physikalischen Eigenschaften des Knochenzements, insbesondere die Schlagzähigkeit, aus.

Der Erfindung liegt daher die Aufgabe zugrunde, einen Kit bereitzustellen, der die Herstellung von weitestgehend blasenfreiem Knochenzement mit hoher Schlagzähigkeit ermöglicht.

Eine weitere Aufgabe besteht darin, eine Paste bereitzustellen, aus der Knochenzement geformt werden kann, der weitestgehend blasenfrei ist und eine hohe Schlagzähigkeit aufweist.

Diese Aufgaben werden gelöst durch die Gegenstände der unabhängigen Ansprüche.

### Demnach stellt die Erfindung einen Kit bereit, umfassend

eine Kitkomponente (a), die als Bestandteile wenigstens (a1) einen monofunktionellen, hydrophoben Methacrylsäureester, (a2) einen Füllstoff und (a3) einen in (a1) löslichen Radikalinitiator, der wenigstens eine Peroxidgruppe aufweist, enthält, wobei Kitkomponente (a) 15- 85 Gewichtsprozent Methacrylsäureester (a1) und weniger als 85 Gewichtsprozent Füllstoffe (a2), bezogen auf das Gesamtgewicht der in Kitkomponente (a) enthaltenen Bestandteile aufweist,

eine Kitkomponente (b), die als Bestandteile wenigstens (b1) einen monofunktionellen, hydrophoben Methacrylsäureester, (b2)einen Füllstoff und (b3) einen in (b1) löslichen Radikalinitiator, der keine Peroxidgruppe aufweist, enthält, wobei Kitkomponente (b) 15 ― 85 Gewichtsprozent Methacrylsäureester (b1) und weniger als 85 Gewichtsprozent Füllstoffe (b2), bezogen auf das Gesamtgewicht der in Kitkomponente (b) enthaltenen Bestandteile aufweist,

eine Kitkomponente (c), die als Bestandteile wenigstens (c1) einen monofunktionellen, hydrophoben Methacrylsäureester, (c2) einen Füllstoff und (c3) einen in (c1) löslichen Akzelerator, der in der Lage ist, aus den Radikalinitiatoren (a3) und (b3) Radikale zu bilden, enthält, wobei Kitkomponente (c) 15 ― 85 Gewichtsprozent Methacrylsäureester (c1) und weniger als 85 Gewichtsprozent Füllstoffe (c2), bezogen auf das Gesamtgewicht der in Kitkomponente (c) enthaltenen Bestandteile aufweist,

wobei wenigstens eine der Kitkomponenten (a), (b) oder (c) wenigstens ein Halogenidsalz enthält und

wenigstens eine der Kitkomponenten (a), (b) oder (c) wenigstens einen Vernetzer enthält,

wobei, bezogen auf das Gesamtgewicht der in den Kitkomponenten (a), (b) und (c) enthaltenen Bestandteile, (i) der Gesamtgewichtsanteil an den Methacrylsäureestern (a1), (b1) und (c1) im Bereich von 15 ― 50 Gewichtsprozent, (ii) der Gesamtgewichtsanteil an den Füllstoffen (a2), (b2) und (c2) im Bereich von 40 - 85 Gewichtsprozent, (iii) der Gewichtsanteil am Radikalinitiator (a3) im Bereich von 0,01 - 4 Gewichtsprozent, (iv) der Gewichtsanteil am Radikalinitiator (b3) im Bereich von 0,01 ― 4 Gewichtsprozent, (v) der Gewichtsanteil am Akzelerator (c3) im Bereich von 0,000001 ― 3 Gewichtsprozent, (vi) der Gesamtgewichtsanteil an dem wenigstens einen Halogenidsalz im Bereich von 0,001 ― 5 Gewichtsprozent und (vii) der Gesamtgewichtsanteil an dem wenigstens einen Vernetzer im Bereich von 0,2 ― 3 Gewichtsprozent liegt.

Ferner stellt die Erfindung eine Paste zur Verfügung, umfassend (i) 15 ― 50 Gewichtsprozent wenigstens eines monofunktionellen, hydrophoben Methacrylsäureesters, (ii) 40 ― 85 Gewichtsprozent wenigstens eines Füllstoffs, (iii) 0,01 ― 4 Gewichtsprozent wenigstens eines im Methacrylsäureester (i) löslichen Radikalinitiators, der wenigstens eine Peroxidgruppe aufweist, (iv) 0,01 ― 4 Gewichtsprozent wenigstens eines im Methacrylsäureester (i) löslichen Radikalinitiators, der keine Peroxidgruppe aufweist, (v) 0,000001 ― 3 Gewichtsprozent wenigstens eines im Methacrylsäureester (i) löslichen Akzelerators, der in der Lage ist, aus den Radikalinitiatoren gemäß (iii) und (iv) Radikale zu bilden, (vi) 0,001 ― 5 Gewichtsprozent wenigstens eines Halogenidsalzes und (vii) 0,2 ― 3 Gewichtsprozent wenigstens eines Vernetzers.

Durch die erfindungsgemäße Kombination des Systems aus voneinander verschiedenen Radkalinitiatoren und dem Akzelerator einerseits sowie dem Vernetzer andererseits gelingt die Herstellung eines nahezu blasenfreien Knochenzementes mit hoher Schlagzähigkeit, der seine Endfestigkeit innerhalb weniger Minuten erreicht.

Wenn, wie aus der DE 102007050762 B3 bekannt, zur Polymerisation der in der Knochenzementpaste enthaltenen Monomere als Initiator entweder ein Barbitursäurederivat oder ein Peroxid zusammen mit einem Kupfersalz eingesetzt wird, dann kommt es zu einer Verringerung der Schlagzähigkeit durch Blasenbildung und unvollständiger Umsetzung der in der Knochenzementpaste enthaltenen Monomere:

Überraschenderweise wurde gefunden, dass die Schlagzähigkeit des Knochenzements erhöht werden kann, wenn einerseits ein Initiatorsystem verwendet wird, das wenigstens einen Radikalinitiator, der eine Peroxidgruppe aufweist, einen Radikalinitiator, der keine Peroxidgruppe aufweist, sowie einen geeigneten Akzelerators enthält, und andererseits die Polymerisation in Gegenwart einer bestimmten Konzentration eines Vernetzers erfolgt.

Diese Effekte sind möglicherweise darauf zurückzuführen, dass es durch die Verwendung des Radikalinitiators, der keine Peroxidgruppe aufweist, zu einem schnellen Einsatz der Polymerisation und einer gleichmäßigen Erwärmung kommt. Im Anschluss daran setzt eine Erstarrung ein, bis durch den wenigstens eine Peroxidgruppe enthaltenden Radikalinitiator eine Nachhärtung eingeleitet wird. Diese Nachhärtung wird von einem starken Hitzestoß begleitet. Ferner scheint es auch zu einer Reaktion zwischen den beiden verschiedenen Radikalinitiatoren zu kommen, die dazu führt, dass die Polymerisation vollständig abläuft. Durch die gleichzeitige Anwesenheit des Vernetzers kommt es zu dabei zu einer massiven, in einem frühen Stadium der Aushärtung einsetzenden Viskositätserhöhung.

Erfindungsgemäß ist es wesentlich, dass der Redoxinitiator, der wenigstens eine Peroxidgruppe aufweist, vor dem Vermischen der Kitkomponenten von dem Redoxinitiator, der keine Peroxidgruppe aufweist, getrennt ist. Würden beide Redoxinitiatoren zusammen in einer Kitkomponente vorliegen, käme es aufgrund einer Reaktion zwischen den beiden Redoxinitiatoren zur Bildung von Radikalen und damit zu einer unerwünschten Polymerisation der vorliegenden Monomere, so dass diese Kitkomponente nicht lagerstabil wäre. Somit sind wenigstens drei Kitkomponenten erforderlich, die getrennt voneinander (i) einen Redoxinitiator, der eine Peroxidgruppe aufweist, (ii) einen Redoxinitiator, der keine Peroxidgruppe aufweist, und (iii) einen Akzelerator enthalten.

Erfindungsgemäß wird somit ein Kit bereitgestellt. Im Rahmen der Erfindung wird unter Kit ein System verstanden, das mehrere separat abgepackte Kitkomponenten aufweist. Die einzelnen Kitkomponenten können beispielsweise steril abgepackt in Flaschen oder Beuteln vorliegen.

Vorliegend umfasst der Kit wenigstens die drei Kitkomponenten (a), (b) und (c), die unterschiedliche Bestandteile enthalten und somit unterschiedliche Zusammensetzungen aufweisen. Es ist erfindungsgemäß auch möglich, dass der Kit mehr als drei Kitkomponenten enthält. Durch Vermischen der in den wenigstens drei Kitkomponenten enthaltenen Zusammensetzungen kann zunächst eine Paste hergestellt werden, die schließlich vom Anwender geformt werden kann, um nach dem Aushärten Knochenzement zu bilden.

Kitkomponente (a) enthält als Bestandteile wenigstens (a1) einen monofunktionellen, hydrophoben Methacrylsäureester, (a2) einen Füllstoff und (a3) einen in (a1) löslichen Radikalinitiator, der wenigstens eine Peroxidgruppe aufweist, wobei Kitkomponente (a) 15 ― 85 Gewichtsprozent Methacrylsäureester (a1) und weniger als 85 Gewichtsprozent Füllstoffe (a2), bezogen auf das Gesamtgewicht der in Kitkomponente (a) enthaltenen Bestandteile aufweist.

Daneben kann Kitkomponente (a) weitere Bestandteile umfassen. Es ist jedoch auch möglich, dass Kitkomponente (a) aus den vorstehend genannten Bestandteilen besteht.

Als monofunktioneller, hydrophober Methacrylsäureester (a1) kommen alle hydrophoben Ester der Methacrylsäure in Betracht.

Durch die Verwendung von hydrophoben monofunktionellen Methacrylsäureestern (a1) kann eine spätere Volumenvergrößerung des Knochenzements und damit eine Beschädigung des Knochens verhindert werden. Gemäß einer bevorzugten Ausführungsform ist der monofunktionelle Methacrylsäureester (a1) hydrophob, wenn er neben der Estergruppe keine weiteren polaren Gruppen enthält. Vorzugsweise weist der monofunktionelle, hydrophobe Methäcrylsäureester (a1) keine Carboxylgruppen, Hydroxylgruppen, Amidgruppen, Sulfonsäuregruppen, Sulfatgruppen, Phosphatgruppen oder Phosphonatgruppen auf.

Die erfindungsgemäß eingesetzten Methacrylsäureester (a1) weisen vorzugsweise eine gewichtsmittlere Molmasse von weniger als 1000 g/mol auf.

Die Angabe der Molmasse bezieht sich im Rahmen der Erfindung auf die viskosimetrisch bestimmte Molmasse.

Bei den Estern handelt es sich vorzugsweise um Alkylester. Die Alkylester sind gemäß einer bevorzugten Ausführungsform Ester von Methacrylsäure mit Alkoholen, die 1 ― 20 Kohlenstoffatome, mehr bevorzugt 1 ― 10 Kohlenstoffatome, noch mehr bevorzugt 1 ― 6 Kohlenstoffatome und ganz besonders bevorzugt 1 ― 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt

Gemäß einer besonders bevorzugten Ausführungsform handelt es sich bei dem monofunktionellen, hydrophoben Methacrylsäureester (a1) um Methacrylsäuremethylester und Methacrylsäureethylester.

Kitkomponente (a) enthält 15 ― 85 Gewichtsprozent, vorzugsweise 20 ― 70 Gewichtsprozent, mehr bevorzugt 25 ― 60 Gewichtsprozent und noch mehr bevorzugt 25 ― 50 Gewichtsprozent wenigstens eines monofunktionellen, hydrophoben Methacrylsäureesters (a1), bezogen auf das Gesamtgewicht der in Kitkomponente (a) enthaltenen Bestandteile. Demnach kann Kitkomponente (a) einen oder mehrere strukturell verschiedene monofunktionelle, hydrophobe Methacrylsäureester (a1) enthalten, solange das Gesamtgewicht der monofunktionellen, hydrophoben Methacrylsäureester (a1) im angegebenen Bereich liegt.

Bei dem in Kitkomponente (a) enthaltenen wenigstens einen Füllstoff (a2) handelt es sich um einen bei Raumtemperatur festen Stoff, der in der Lage ist, die Viskosität der aus den übrigen in Kitkomponente (a) enthaltenen-Bestandteilen zusammengesetzten Mischung zu erhöhen. Der Füllstoff (a2) muss biokompatibel sein.

Gemäß einer bevorzugten Ausführungsform ist der Füllstoff (a2) aus der Gruppe ausgewählt, die aus (i) in wenigstens dem oder einem der Methacrylsäureester (a1), (b1) und (c1) löslichen Polymer, mehr bevorzugt in wenigstens dem oder einem der Methacrylsäureester (a1) löslichen Polymer, (ii) in wenigstens dem oder einem der Methacrylsäureester (a1), (b1) und (c1) unlöslichen Polymer, mehr bevorzugt in wenigstens dem oder einem der Methacrylsäureester (a1) unlöslichen Polymer, (iii) anorganischen Salzen, (iv) anorganischen Oxiden, (v) Metallen und (vi) Metalllegierungen besteht. Der Füllstoff (a2) ist vorzugsweise partikulär. Gemäß einer besonders bevorzugten Ausführungsform weist der Füllstoff (a2) eine durchschnittliche Teilchengröße im Bereich von 10 nm - 100 µm und besonders bevorzugt im Bereich von 100 nm -10 µm auf. Unter durchschnittlicher Teilchengröße wird vorliegend ein Größenbereich verstanden, der von wenigstens 90 Prozent der Teilchen eingenommen wird.

Im Rahmen der Erfindung werden unter dem Begriff, Polymere sowohl Homopolymere als auch Copolymere zusammengefasst.

Bei dem in wenigstens einem der Methacrylsäureester (a1), (b1) und (c1) löslichen Polymer handelt es sich vorzugsweise um ein Polymer mit einer gewichtsmittleren Molmasse von wenigstens 150.000 g/mol. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAA), Polymethacrylsäurepropylester (PMAP), Polymethacrylsäureisopropylester und Polymethyl-co-acrylmethacrylat besteht.

Bei dem in wenigstens einem der Methacrylsäureester (a1), (b1) und (c1) unlöslichen Polymer handelt es sich beispielsweise um Polyethylen, Polypropylen oder Polybutadien. Das in wenigstens einem der Methacrylsäureester (a1), (b1) und (c1) unlösliche Polymer kann vernetzt oder nicht vernetzt sein.

Das als Füllstoff (a2) verwendbare anorganische Salz kann ein im Methacrylsäureester (a1) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (a2) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (a2) verwendbaren Metall kann es sich beispielsweise um ein Übergangsmetall handeln. Gemäß einer bevorzugten Ausführungsform ist das Metall Tantal oder Wolfram.

Die als Füllstoff (a2) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Anteil des wenigstens einen Füllstoffs (a2) beträgt weniger als 85 Gewichtsprozent, vorzugsweise weniger als 80 Gewichtsprozent und mehr bevorzugt weniger als 75 Gewichtsprozent, bezogen auf das Gesamtgewicht der in Kitkomponente (a) enthaltenen Bestandteile. Vorzugsweise enthält Kitkomponente (a) 15 - 84,99 Gewichtsprozent, mehr bevorzugt 15 - 80 Gewichtsprozent und noch mehr bevorzugt 20 ― 75 Gewichtsprozent an dem wenigstens einen Füllstoff (a2), bezogen auf das Gesamtgewicht der in Kitkomponente (a) enthaltenen Bestandteile. Dementsprechend kann Bestandteil (a) einen oder mehrere strukturell verschiedene Füllstoffe (a2) enthalten, solange das Gesamtgewicht der Füllstoffe (a2) im angegebenen Bereich liegt.

Bestandteil (a) enthält außerdem einen in dem monofunktionellen, hydrophobem Methacrylsäureester (a1) löslichen Radikalinitiator (a3), der wenigstens eine Peroxidgruppe aufweist. Obwohl hierin in der Singularform verwendet, erstreckt sich der Begriff "Radikalinitiator (a3)" erfindungsgemäß auch auf eine Mehrzahl an strukturell verschiedenen Radikalinitiatoren (a3).

Unter Radikalinitiator (a3) wird vorliegend eine Verbindung verstanden, aus der durch Einwirkung des Akzelerators (c3) ein Radikal gebildet werden kann, das in der Lage ist, die Polymerisation der Methacrylsäureester (a1), (a2) und (a3) auszulösen. Bei dem Radikalinitiator (a3) handelt es sich somit um einen radikalischen Polymerisationsstarter.

Der Radikalinitiator (a3) weist in Gegenwart des Akzelerators (c3) eine vom Radikalinitiator (b3) verschiedene Zerfallsgeschwindigkeit auf. Gemäß einer besonders bevorzugten Ausführungsform weist der der Radikalinitiator (a3) in Gegenwart des Akzelerators (c3) eine niedrigere Zerfallsgeschwindigkeit auf als der Radikalinitiator (b3).

Gemäß einer bevorzugten Ausführungsform beträgt die Löslichkeit des Radikalinitiators (a3) im Methacrylsäureester (a1) wenigstens 0,5 Gewichtsprozent, bezogen auf das Gewicht des Methacrylsäureesters (a1).

Gemäß einer besonders bevorzugten Ausführungsform ist der Radikalinitiator (a3) aus der Gruppe ausgewählt, die aus Dibenzoylperoxid und Dilauroylperoxid besteht.

Bestandteil (a) enthält vorzugsweise 0,01 - 12 Gewichtsprozent, vorzugsweise 0,01 - 10 Gewichtsprozent, mehr bevorzugt 0,05 ― 8 Gewichtsprozent und noch mehr bevorzugt 0,05 - 5 Gewichtsprozent wenigstens eines Radikalinitiators (a3), bezogen auf das Gesamtgewicht der in Kitkomponente (a) enthaltenen Bestandteile.

Kitkomponente (b) enthält als Bestandteile wenigstens (b1) einen monofunktionellen, hydrophoben Methacrylsäureester, (b2) einen Füllstoff und (b3) einen, in (b1) löslichen Radikalinitiator, der keine Peroxidgruppe aufweist, wobei Kitkomponente (b) 15 - 85 Gewichtsprozent Methacrylsäureesters (b1) und weniger als 85 Gewichtsprozent Füllstoffe (b2), bezogen auf das Gesamtgewicht der in Kitkomponente (b) enthaltenen Bestandteile aufweist. Daneben kann Bestandteil (b) weitere Komponenten umfassen. Es ist jedoch auch möglich, dass Bestandteil (b) aus den vorstehend genannten Komponenten besteht.

Als monofunktioneller, hydrophober Methacrylsäureester (b1) kommen alle hydrophoben Ester der Methacrylsäure in Betracht.

Durch die Verwendung von hydrophoben monofunktionellen Methacrylsäureestern (b1) kann eine spätere Volumenvergrößerung des Knochenzements und damit eine Beschädigung des Knochens verhindert werden. Gemäß einer bevorzugten Ausführungsform ist der monofunktionelle Methacrylsäureester (b1) hydrophob, wenn er neben der Estergruppe keine weiteren polaren Gruppen aufweist. Vorzugsweise weist der monofunktionelle, hydrophobe Methacrylsäureester (b1) keine Carboxylgruppen, Hydroxylgruppen, Amidgruppen, Sulfonsäuregruppen, Sulfatgruppen, Phosphatgruppen oder Phosphonatgruppen auf.

Die erfindungsgemäß eingesetzten Methacrylsäureester (b1) weisen vorzugsweise eine gewichtsmittlere Molmasse von weniger als 1000 g/mol auf.

Bei den Estern handelt es sich vorzugsweise um Alkylester.Die Alkylester sind gemäß einer bevorzugten Ausführungsform Ester von Methacrylsäure mit Alkoholen, die 1 ― 20 Kohlenstoffatome, mehr bevorzugt 1 ― 10 Kohlenstoffatome, noch mehr bevorzugt 1 ― 6 Kohlenstoffatome und ganz besonders bevorzugt 1 ― 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem monofunktionellen, hydrophoben Methacrylsäureester (b1) um Methacrylsäuremethylester oder Methacrylsäureethylester.

Kitkomponente (b) enthält 15 ― 85 Gewichtsprozent, vorzugsweise 20 ― 70 Gewichtsprozent, mehr bevorzugt 25 ― 60 Gewichtsprozent und noch mehr bevorzugt 25 ― 50 Gewichtsprozent wenigstens eines monofunktionellen, hydrophoben Methacrylsäureesters (b1), bezogen auf das Gesamtgewicht der in Kitkomponente (b) enthaltenen Bestandteile. Demnach kann Kitkomponente (b) einen oder mehrere strukturell verschiedene monofunktionelle, hydrophobe Methacrylsäureester (b1) enthalten, solange das Gesamtgewicht der monofunktionellen, hydrophoben Methacrylsäureester (b1) im angegebenen Bereich liegt.

Bei dem in Kitkomponente (b) enthaltenen wenigstens einen Füllstoff (b2) handelt es sich um einen bei Raumtemperatur festen Stoff, der in der Lage ist, die Viskosität der aus den übrigen in Kitkomponente (b) enthaltenen Bestandteilen zusammengesetzten Mischung zu erhöhen. Der Füllstoff (b2) muss biokompatibel sein.

Gemäß einer bevorzugten Ausführungsform ist der Füllstoff (b2) aus der Gruppe ausgewählt, die aus (i) in wenigstens dem oder einem der Methacrylsäureester (a1), (b1) und (c1) löslichen Polymeren, mehr bevorzugt in wenigstens dem oder einem der Methacrylsäureester (b1) löslichen Polymeren, (ii) in wenigstens dem oder einem der Methacryisäureester,(a1), (b1) und (c1) unlöslichen Polymeren, mehr bevorzugt in wenigstens dem oder einem der Methacrylsäureester (b1) unlöslichen Polymeren, (iii) anorganischen Salzen, (iv) anorganischen Oxiden, (v) Metallen und (vi) Metalllegierungen besteht.

Bei dem in wenigstens einem der Methacrylsäureester (a1), (b1) und (c1) löslichen Polymer handelt es sich vorzugsweise um ein Polymer mit einer gewichtsmittleren Molmasse von wenigstens 150.000 g/mol. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAA), Polymethacrylsäurepropylester (PMAP), Polymethacrylsäureisopropylester und Polymethyl-co-acrylmethacrylat besteht

Bei dem in wenigstens einem der Methacrylsäureester (a1), (b1) und (c1) unlöslichen Polymer handelt es sich beispielsweise um Polyethylen, Polypropylen oder Polybutadien. Das in wenigstens einem der Methacrylsäureester (a1), (b1) und (c1) unlösliche Polymer kann vernetzt oder nicht vernetzt sein.

Das als Füllstoff (b2) verwendbare anorganische Salz kann ein im Methacrylsäureester (b1) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführurigsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (b2) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (b2) verwendbaren Metall kann es sich beispielsweise um ein Übergangsmetall handeln. Gemäß einer bevorzugten Ausführungsform ist das Metall Tantal oder Wolfram.

Die als Füllstoff (b2) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Anteil des wenigstens einen Füllstoffs (b2) beträgt weniger als 85 Gewichtsprozent, vorzugsweise weniger als 80 Gewichtsprozent und mehr bevorzugt weniger als 75 Gewichtsprozent, bezogen auf das Gesamtgewicht der in Kitkomponente (b) enthaltenen Bestandteile. Vorzugsweise enthält Kitkomponente (b) 15 ― 84,99 Gewichtsprozent, mehr bevorzugt 15―80 Gewichtsprozent und noch mehr bevorzugt 20 ― 75 Gewichtsprozent an dem wenigstens einen Füllstoff (b2), bezogen auf das Gesamtgewicht der in Kitkomponente (b) enthaltenen Bestandteile. Demnach kann Bestandteil (b) einen oder mehrere strukturell verschiedene Füllstoffe (b2) enthalten, solange das Gesamtgewicht der Füllstoffe (b2) im angegebenen Bereich liegt.

Bestandteil (b) enthält außerdem einen in dem monofunktionellen, hydrophoben Methacrylsäureester (b1) löslichen Radikalinitiator (b3), der keine Peroxidgruppe aufweist. Obwohl hierin in der Singularform verwendet, erstreckt sich der Begriff "Radikalinitiator (b3)" erfindungsgemäß auch auf eine Mehrzahl an strukturell verschiedenen Radikalinitiatoren (b3).

Unter Radikalinitiator (b3) wird vorliegend eine Verbindung verstanden, aus der durch Einwirkung des Akzelerators (c3) ein Radikal gebildet werden kann, das in der Lage ist, die Polymerisation der Methacrylsäureester (b1), (b2) und (b3) auszulösen. Bei dem Radikalinitiator (b3) handelt es sich somit um einen radikalischen Polymerisationsstarter.

Der Radikalinitiator (b3) weist in Gegenwart des Akzelerators (c3) eine vom Radikalinitiator (a3) verschiedene Zerfallsgeschwindigkeit auf. Gemäß einer besonders bevorzugten Ausführungsform weist der der Radikalinitiator (b3) in Gegenwart des Akzelerators (c3) eine höhere Zerfallsgeschwindigkeit auf als der Radikalinitiator (a3).

Gemäß einer bevorzugten Ausführungsform beträgt die Löslichkeit des Radikalinitiators (b3) im Methacrylsäureester (b1) wenigstens 0,5 Gewichtsprozent, bezogen auf das Gewicht des Methacrylsäureesters (b1).

Bei dem Radikalinitiator (b3) handelt es sich vorzugsweise um Barbitursäure oder Barbitursäurederivate, bei denen die Barbitursäure an wenigstens einer der Positionen 1 oder 5 einen Substituenten trägt. Solche Barbitursäurederivate weisen den Vorteil auf, dass sie keine pharmakologische Wirkung zeigen.

Das Barbitursäurederivat ist erfindungsgemäß vorzugsweise monosubstituiert. Gemäß einer bevorzugten Ausführungsform wird als Radikalinitiator (b3) eine an Position 5 substituierte Barbitursäure eingesetzt. Bei dem Substituenten des Barbitursäurederivats handelt es sich vorzugsweise ferner um einen hydrophoben Substituenten. Vorzugsweise handelt es sich bei dem Barbitursäurederivat um ein Alkyl-, Cycloalkyl- oder Arylderivat der Barbitursäure.

Gemäß einer besonders bevorzugten Ausführungsform ist das Barbitursäurederivat aus der Gruppe ausgewählt, die aus Cyclohexylbarbitursäure, 1,3,5-Trimethylbarbitursäure, 1-Phenyl-5-benzylbarbitursäure, 1-Benzyl-5-phenylbarbitursure, 1,3-Dimethylbarbitursäure, 1,3-Dimethyl-5-phenylbarbitursäure, 1-Cyclohexyl-5-ethylbarbitursäure, 5-Laurylbarbitursäure, 1-n-Butyl-barbitursäure, 5-n-Butylbarbitursäure, 5-Allylbarbitursäure, 5-Hydroxy-5-butylbarbitursäure, 5,5-Dibrombarbitursäure, Trichlorbarbitursäure, 5-Nitrobarbitursäure, 5-Aminobarbitursäure, 5-Hydroxybarbitursäure und 5,5-Dihydroxybarbitursäure besteht.

Unter einer ganz besonders bevorzugten Ausführungsform ist das Barbitursäurederivat aus der Gruppe ausgewählt, die aus 1-Cyclohexyl-5-ethyl-barbitursäure, 1-n-Butyl-barbitursäure und 5-n-Butyl-barbitursäure besteht.

Unter den Begriff Barbitursäurederivate fallen erfindungsgemäß auch Erdalkali- und Alkalisalze dieser Barbitursäurederivate.

Kitkomponente (b) enthält vorzugsweise 0,01 ― 12 Gewichtsprozent, vorzugsweise 0,01 ―8 Gewichtsprozent, mehr bevorzugt 0,05 ―6 Gewichtsprozent und noch mehr bevorzugt 0,05― 5 Gewichtsprozent wenigstens eines Radikalinitiators (b3), bezogen auf das Gesamtgewicht der in Kitkomponente (b) enthaltenen Bestandteile. Demnach kann Kitkomponente (b) einen oder mehrere strukturell verschiedene Radikalinitiatoren (b3) enthalten, solange das Gesamtgewicht des Radikalinitiators (b3) im angegebenen Bereich liegt.

Kitkomponente (b) enthält als Bestandteile wenigstens (c1) einen monofunktionellen, hydrophoben Methacrylsäureester, (c2) einen Füllstoff und (c3) einen in (c1) löslichen Akzelerator, der in der Lage ist, aus den Radikalinitiatoren (a3) und (b3) Radikale zu bilden, wobei Kitkomponente (c) 15 ― 85 Gewichtsprozent Methacrylsäureester (c1) und weniger als 85 Gewichtsprozent Füllstoffe (c2), bezogen auf das Gesamtgewicht der in Kitkomponente (c) enthaltenen Bestandteile aufweist.

Als monofunktioneller, hydrophober Methacrylsäureester (c1) kommen alle hydrophoben Ester der Methacrylsäure in Betracht.

Durch die Verwendung von hydrophoben monofunktionellen Methacrylsäureesterri (c1) kann eine spätere Volumenvergrößerung des Knochenzements und damit eine Beschädigung des Knochens verhindert werden. Gemäß einer bevorzugten Ausführungsform ist der monofunktionelle Methacrylsäureester (c1) hydrophob, wenn er neben der Estergruppe keine weiteren polaren Gruppen aufweist. Vorzugsweise weist der monofunktionelle, hydrophobe Methacrylsäureester (c1) keine Carboxylgruppen, Hydroxylgruppen, Amidgruppen, Sulfonsäuregruppen, Sulfatgruppen, Phosphatgruppen oder Phosphonatgruppen auf.

Die erfindungsgemäß eingesetzten Methacrylsäureester (c1) weisen vorzugsweise eine gewichtsmittlere Molmasse von weniger als 1000 g/mol auf.

Bei den Estern handelt es sich vorzugsweise um Alkylester. Die Alkylester sind gemäß einer bevorzugten Ausführungsform Ester von Methacrylsäure mit Alkoholen, die 1 ― 20 Kohlenstoffatome, mehr bevorzugt 1 ― 10 Kohlenstoffatome, noch mehr bevorzugt 1 ― 6 Kohlenstoffatome und ganz besonders bevorzugt 1 ― 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem monofunktionellen, hydrophoben Methacrylsäureester (c1) um Methacrylsäuremethylester oder Methacrylsäureethylester.

Kitkomponente (c) enthält 15 ― 85 Gewichtsprozent, vorzugsweise 20 ― 70 Gewichtsprozent, mehr bevorzugt 25 ― 60 Gewichtsprozent und noch mehr bevorzugt 25 ― 50 Gewichtsprozent wenigstens eines monofunktionellen, hydrophoben Methacrylsäureesters (c1), bezögen auf das Gesamtgewicht der in Kitkomponente (c) enthaltenen Bestandteile. Demnach kann Kitkomponente (c) einen oder mehrere strukturell verschiedene monofunktionelle, hydrophobe Methacrylsäureester (c1) enthalten, solange das Gesamtgewicht der monofunktionellen, hydrophoben Methacrylsäureester (c1) im angegebenen Bereich liegt.

Bei dem in Kitkomponente (c) enthaltenen wenigstens einen Füllstoff (c2) handelt es sich um einen bei Raumtemperatur festen Stoff, der in der Lage ist, die Viskosität der aus den übrigen in Kitkomponente (c) enthaltenen Bestandteilen zusammengesetzten Mischung zu erhöhen. Der Füllstoff (c2) muss biokompatibel sein.

Gemäß einer bevorzugten Ausführungsform ist der Füllstoff (c2) aus der Gruppe ausgewählt, die aus (i) in wenigstens dem oder einem der Methacrylsäureester (a1), (b1) und (c1) löslichen Polymeren, mehr bevorzugt in wenigstens dem oder einem der Methacrylsäureester (c1) löslichen Polymeren, (ii) in wenigstens dem oder einem der Methacrylsäureester (a1), (b1) und (c1) unlöslichen Polymeren, mehr bevorzugt in wenigstens dem oder einem der Methacrylsäureester (c1) unlöslichen Polymeren, (iii) anorganischen Salzen, (iv) anorganischen Oxiden, (v) Metallen und (vi) Metalllegierungen besteht.

Bei dem in wenigstens einem der Methacrylsäureester (a1), (b1) und (c1) löslichen Polymer handelt es sich vorzugsweise um ein Polymer mit einer gewichtsmittleren Molmasse von wenigstens 150.000 g/mol. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polyrüethacrylsäuremethylester (PMMA), Polymethacrylsäureethylester (PMAA), Polymethacrylsäurepropylester (PMAP), Polymethacrylsäureisopropylester und Polymethyl-co-acrylmethacrylat besteht.

Bei dem in wenigstens einem der Methacrylsäureester (a1), (b1) und (c1) unlöslichen Polymer handelt es sich beispielsweise um Polyethylen, Polypropylen oder Polybutadien. Das in wenigstens einem der Methacrylsäureester (a1), (b1) und (c1) unlösliche Polymer kann vernetzt oder nicht vernetzt sein.

Das als Füllstoff (c2) verwendbare anorganische Salz kann ein im Methacrylsäureester (c1) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (c2) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (c2) verwendbaren Metall kann es sich beispielsweise um ein Übergangsmetall handeln. Gemäß einer bevorzugten Ausführungsform ist das Metall Tantal oder Wolfram.

Die als Füllstoff (c2) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Anteil des wenigstens einen Füllstoffs (c2) beträgt weniger als 85 Gewichtsprozent, vorzugsweise weniger als 80 Gewichtsprozent und mehr bevorzugt weniger als 75 Gewichtsprozent, bezogen auf das Gesamtgewicht der in Kitkomponente (c) enthaltenen Bestandteile. Vorzugsweise enthält Kitkomponente (c) 15 ―84,99 Gewichtsprozent, mehr bevorzugt 15 ― 80 Gewichtsprozent und noch mehr bevorzugt 20 ― 75 Gewichtsprozent an dem wenigstens einen Füllstoff (c2), bezogen auf das Gesamtgewicht der in Kitkomponente (c) enthaltenen Bestandteile. Demnach kann Bestandteil (c) einen oder mehrere strukturell verschiedene Füllstoffe (c2) enthalten, solange das Gesamtgewicht der Füllstoffe (c2) im angegebenen Bereich liegt.

Kitkomponente (c) enthält außerdem einen in dem monofunktionellen, hydrophoben Methacrylsäureester (c1) löslichen Akzelerator (c3), der in der Lage ist, aus den Radikalinitiatoren (a3) und (b3) Radikale zu bilden. Obwohl hierin in der Singularform verwendet, erstreckt sich der Begriff "Akzelerator (c3)" erfindungsgemäß auch auf eine Mehrzahl an strukturell verschiedenen Akzeleratoren (c3).

Gemäß einer bevorzugten Ausführungsform beträgt die Löslichkeit des Akzelerators (c3) im Methacrylsäureester (c1) wenigstens 0,5 Gewichtsprozent, bezogen auf das Gewicht des Methacrylsäureesters (c1).

Unter Akzelerator, der in der Lage ist, aus den Radikalinitiatoren (a3) und (b3) Radikale zu bilden, werden Verbindungen verstanden, die die Radikalinitiatoren (a3) und (b3) gegebenenfalls in Gegenwart weiterer, im erfindungsgemäßen Kit enthaltenen Verbindungen, wie beispielsweise Halogenidionen, zu Radikalen umsetzen können. Solche Akzeleratoren sind aus dem Stand der Technik gut bekannt.

Bei dem Akzelerator (c3) handelt es sich vorzugsweise um ein Salz mit Ionen von Metallen, die neben der Oxidationsstufe 0 wenigstens zwei weitere Oxidationsstufen einnehmen können. Gemäß einer besonders bevorzugten Ausführungsform sind die Metallionen aus der Gruppe ausgewählt, die aus Kupferionen, Eisenionen, Cobaltionen und Manganionen besteht. Demnach wird als Akzelerator (c3) vorzugsweise ein Eisensalz, ein Cobaltsalz oder ein Mangansalz eingesetzt. Gemäß einer ganz besonders bevorzugten Ausführungsform ist der Akzelerator (c3) aus der Gruppe ausgewählt, die aus Kupfer(II)-2-ethylhexanoat, Kupfer(II)-laurat, Kupfer(II)-decanoat, Kupfer(II)-octoat, Kupfer(II)acetylacetonat und Kupfer(II)-methacrylat besteht.

Kitkomponente (c) enthält vorzugsweise 0,00001 ― 12 Gewichtsprozent, vorzugsweise 0,0001 - 9 Gewichtsprozent, mehr bevorzugt 0,001 ―6 Gewichtsprozent und noch mehr bevorzugt 0,05 ― 5 Gewichtsprozent wenigstens eines Akzelerators (c3), bezogen auf das Gesamtgewicht der in Kitkomponente (c) enthaltenen Bestandteile. Demnach kann Kitkomponente (c) einen oder mehrere strukturell verschiedene Akzeleratoren (c3) enthalten, solange das Gesamtgewicht des Akzelerators (c3) im angegebenen Bereich liegt.

Wenigstens eine der Kitkomponenten (a), (b) oder (c) enthält ferner ein Halogenidsalz.

Bei dem Halogenidsalz handelt es sich vorzugsweise um ein in wenigstens einem der Methacrylsäureester (a1), (b1) und (c1) lösliches Halogenidsalz. Vorzugsweise ist das Halogenidsalz in wenigstens einem der Methacrylsäureester (a1), (b1) und (c1) löslich, mit dem das Halogenidsalz zusammen in der Kitkomponente vorliegt.

Gemäß einer bevorzugten Ausführungsform beträgt die Löslichkeit des Halogenidsalzes in wenigstens einem der Methacrylsäureester (a1), (b1) und (c1) wenigstens 0,5 Gewichtsprozent, bezogen auf das Gewicht des wenigstens einen Methacrylsäureesters (a1), (b1) und (c1).

Erfindungsgemäß werden als Halogenidsalze Salze verstanden, die wenigstens eine Art von Halogenidionen enthalten und in wenigstens einem der Methacrylsäureester (a1), (b1) und (c1) dissoziieren. Vorzugsweise handelt es sich bei den Halogenidionen um, Chloridionen oder Bromidionen, ganz bevorzugt um Chloridionen.

Erfindungsgemäß können als Halogenidsalze vorzugsweise Metallhalogenide, Hydrochloride und quarternäre Ammoniumhalogenidsalze eingesetzt werden.

Gemäß einer besonders bevorzugten Ausführungsform ist das Halogenidsalz aus der Gruppe ausgewählt, die aus Kupfer(II)-chlorid, Kupfer (II)-bromid, Eisen(III)-chlorid, Eisen(III)-bromid, Kobalt(II)-chlorid, Kobalt(II)-bromid, Trieihylaminhydrochlorid, Triethylaminhydrobromid, Propylaminhydrochlorid, Butylaminhydrochlorid, Methacryloylcholinchlorid, Trioctylmethylammoniumchlorid und Triethylbenzylammoniumchlorid besteht.

Der Anteil des wenigstens einen Halogenidsalzes in wenigstens einer der Kitkomponenten (a), (b) oder (c) beträgt vorzugsweise 0,002 ― 10 Gewichtsprozent, vorzugsweise 0,002 ― 7 Gewichtsprozent und mehr bevorzugt 0,003 ― 6 Gewichtsprozent, bezogen auf das Gesamtgewicht der in dieser wenigstens einen Kitkomponente enthaltenen Bestandteile.

Ferner enthält wenigstens eine der Kitkomponente (a), (b) oder (c) einen Vernetzer.

Bei dem Vernetzer handelt es sich um eine bifunktionelle oder trifunktionelle Verbindung.

Erfindungsgemäß soll der Vernetzer eine Vernetzung des bei der Aushärtung des Knochenzementes polymerisierenden monofunktionellen, hydrophoben Methacrylsäureesters bewirken.

Gemäß einer bevorzugten Ausführungsform weist der Vernetzer wenigstens zwei Acrylatgruppen auf.

Besonders bevorzugt ist der Vernetzer aus der Gruppe ausgewählt, die aus Ethylenglycoldimethacrylat, Butylenglycoldimethacrylat (z.B. Butan-1,4-diol-dimethacrylat) und Hexamethylendimethacrylat (z.B. Hexan-1,6-diol-dimethacrylat) besteht.

Der Anteil des wenigstens einen Vernetzers beträgt vorzugsweise 0,3 ― 10 Gewichtsprozent, vorzugsweise 0,4 ― 8 Gewichtsprozent und mehr bevorzugt 0,5 ― 6 Gewichtsprozent, bezogen auf das Gesamtgewicht der in dieser wenigstens einen Kitkomponente enthaltenen Bestandteile.

Der erfindungsgemäße Kit kann ferner wenigstens eine pharmazeutische Substanz enthalten.

Die pharmazeutische Substanz kann vorzugsweise aus der Gruppe ausgewählt sein, die aus Antibiotika, Aniphlogistika, Hormonen, Wachstumsfaktoren, Bisphosphonaten und Cytostatika besteht.

Das wenigstens eine Antibiotikum ist vorzugsweise aus der Gruppe ausgewählt, die aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid, Moxifloxacin, Ciprofloxacin, Teicoplanin, Vancomycin, Ramoplanin, Metronidazol, Tinidazol und Omidazol besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem Fibroblast Growth Factor (FGF), dem Transforming Growth Factor (TGF), dem Platelet Derived Growth Factor (PDGF), dem Epidermalen Wachstumsfaktor (EGF), dem Vascular Endothelial Growth Factor (VEGF), insulinähnlichen Wachstumsfaktoren (IGF), dem Hepatocyte Growth Factor (HGF), Interleukin-1B, Interleukin 8 und dem Nervenwachstumsfaktor besteht. Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

Die wenigstens eine pharmazeutische Substanz kann in einem oder mehreren der Kitkomponenten (a), (b) und (c) enthalten sein.

Erfindungsgemäß kann der Kit ferner einen Röntgenopaker aufweisen.

Der Röntgenopaker ist vorzugsweise aus der Gruppe ausgewählt, die aus Zirkoniumdioxid, Bariumsulfat und Tantal besteht.

Der wenigstens eine Röntgenopaker kann in einem oder mehreren der Kitkomponenten (a), (b) und (c) enthalten sein.

Der Kit kann erfindungsgemäß ferner wenigstens einen Farbstoff enthalten.

Bei dem Farbstoff kann es sich besonders bevorzugt um einen Lebensmittelfarbstoff handeln. Gemäß einer besonders bevorzugten Ausführungsform wird als Farbstoff Chlorophyllin (E141), Riboflavin und/oder Lissamingrün eingesetzt.

Der wenigstens eine Farbstoff kann in einem oder mehreren der Kitkomponenten (a), (b) und (c) enthalten sein.

Der Kit kann außerdem wenigstens einen Stabilisator enthalten.

Der Stabilisator soll geeignet sein, eine Spontanpolymerisation der in den Kitkomponenten (a), (b) und (c) enthaltenen Monomere zu verhindern. Ferner soll der Stabilisator keine störenden Wechselwirkungen mit den anderen in den Kitkomponenten enthaltenen Bestandteilen zeigen. Derartige Stabilisatoren sind aus dem Stand der Technik bekannt.

Gemäß einer bevorzugten Ausführungsform handelt es sich bei dem Stabilisator um 2,6-Di-tert-butyl-4-methylphenol und/oder 2,6-Di-tert-butyl-phenol.

Der wenigstens eine Stabilisator kann in einem oder mehreren der Kitkomponenten (a), (b) und (c) enthalten sein und ist vorzugsweise in jeder der drei Kitkomponenten (a), (b) und (c) enthalten.

Darüber hinaus kann der Kit gegebenenfalls weitere Additive aufweisen.

Aufgrund der definierten Gewichtsanteile an Methacrylsäureestern (a1), (b1) und (c1) und Füllstoffen (a2), (b2) und (c2) liegen die Kitkomponenten (a), (b) und (c) als Pasten vor. Dies hat den Vorteil, dass vom Anwender problemlos eine Knochenzementpaste durch Mischen der verschiedenen pastösen Kitkomponenten hergestellt werden kann. Insbesondere werden dadurch die Nachteile beseitigt, die beim Mischen von Komponenten, die in unterschiedlichen Aggregatzuständen vorliegen, zum Beispiel beim Mischen einer pulverförmigen Komponente und einer flüssigen Monomerkomponente, bestehen.

Die Kitkomponenten (a), (b) und (c) sind so aufeinander abgestimmt, dass die darin enthaltenen, einzelnen Bestandteile bezogen auf das Gesamtgewicht der in den Kitkomponenten enthaltenen Bestandteile in genau definierten Mengenbereichen vorliegen.

Unter Gesamtgewicht der in den Kitkomponenten (a), (b) und (c) enthaltenen Bestandteile wird erfindungsgemäß die Summe der Gewichte der in den Kitkomponenten (a), (b) und (c) enthaltenen Bestandteile verstanden.

Unter Gesamtgewichtsanteil an einem bestimmten Bestandteil, bezogen auf das Gesamtgewicht der in den Kitkomponenten (a), (b) und (c) enthaltenen Bestandteile, wird erfindungsgemäß der Anteil am Gesamtgewicht der in den Kitkomponenten (a), (b) und (c) enthaltenen Bestandteile verstanden, der von der Summe der Gewichtsanteile des in den Kitkomponenten (a), (b) und/oder (c) enthaltenen, bestimmten Bestandteils eingenommen wird:

Wenn sich beispielsweise das Gesamtgewicht der Bestandteile von Kitkomponente (a) auf 100 g, das Gesamtgewicht der Bestandteile von Kitkomponente (b) auf 100 g und das Gesamtgewicht der Bestandteile von Kitkomponente (c) auf 100 g beläuft, so beträgt das Gesamtgewicht der in den Kitkomponenten (a), (b) und (c) enthaltenen Bestandteile 300 g.

Sind gemäß diesem Beispiel in Kitkomponente (a) 30 g Füllstoffe (a2), in Kitkomponente (b) 80 g Füllstoffe (b2) und in Kitkomponente (c) 40 g Füllstoffe (c2) enthalten, so beträgt das Gesamtgewicht an den Füllstoffen (a2), (b2) und (c2) 150 g. Dementsprechend beträgt der Gesamtgewichtsanteil an den Füllstoffen (a2), (b2) und (c2) in diesem Beispiel 50 Gewichtsprozent, bezogen auf das Gesamtgewicht der in den Kitkomponenten (a), (b) und (c) enthaltenen Bestandteile.

Der Gesamtgewichtsanteil an den Methacrylsäureestern (a1), (b1) und (c1) liegt im Bereich von - 15 ― 50 Gewichtsprozent, vorzugsweise 15 ― 45 Gewichtsprozent und mehr bevorzugt 20 ― 45 Gewichtsprozent, bezogen auf das Gesamtgewicht der in den Kitkomponenten, vorzugsweise den Kitkomponenten (a), (b) und (c), enthaltenen Bestandteile.

Der Gesamtgewichtsanteil an den Füllstoffen (a2), (b2) und (c2) liegt im Bereich von 40 ― 85 Gewichtsprozent, vorzugsweise 42 ― 83 Gewichtsprozent, mehr bevorzugt 45 ― 80 Gewichtsprozent und noch mehr bevorzugt 50 ― 75 Gewichtsprozent, bezogen auf das Gesamtgewicht der in den Kitkomponenten, vorzugsweise den Kitkomponenten (a), (b) und (c), enthaltenen Bestandteile.

Durch diesen Anteil an Methacrylsäureestern (a1), (b1) und (c1) und Füllstoffen (a2), (b2) und (c2) ergibt sich nach dem Vermischen der Bestandteile (a), (b) und (c) eine Paste, die vom Anwender leicht zu verarbeiten, insbesondere leicht formbar, ist.

Der Gewichtsanteil am Radikalinitiator (a3) liegt im Bereich von 0,01 ― 4 Gewichtsprozent, vorzugsweise im Bereich von 0,01 - 3 Gewichtsprozent, mehr bevorzugt im Bereich von 0,05 ― 2,5 Gewichtsprozent und noch mehr bevorzugt im Bereich von 0,05 ― 2 Gewichtsprozent, bezogen auf das Gesamtgewicht der in den Kitkomponenten, vorzugsweise den Kitkomponenten (a), (b) und (c), enthaltenen Bestandteile.

Der Gewichtsanteil am Radikalinitiator (b3) liegt im Bereich von 0,01 ― 4 Gewichtsprozent, vorzugsweise im Bereich von 0,01 - 3 Gewichtsprozent, mehr bevorzugt im Bereich von 0,05 - 2,5 Gewichtsprozent und noch mehr bevorzugt im Bereich von 0,05 ― 2 Gewichtsprozent, bezogen auf das Gesamtgewicht der in den Kitkomponenten, vorzugsweise den Kitkomponenten (a), (b) und (c), enthaltenen Bestandteile.

Der Gewichtsanteil am Akzelerator (c3) liegt im Bereich von 0,00001 ― 4 Gewichtsprozent, vorzugsweise 0,0001 ― 3 Gewichtsprozent, mehr bevorzugt 0,001 ― 3 Gewichtsprozent und noch mehr bevorzugt 0,05 ― 2 Gewichtsprozent, bezogen auf das Gesamtgewicht der in den Kitkomponenten, vorzugsweise den Kitkomponenten (a), (b) und (c), enthaltenen Bestandteile.

Die Gewichtsanteile am Radikalinitiator (a3), Radikalinitiator (b3) und Akzelerator (c3) sind nicht besonders kritisch. Ein Gehalt, der geringer ist als die Untergrenze der angegebenen Bereiche führt dazu, dass die Aushärtung des Knochenzements ohne Einsatz weiterer Polymerisationshilfsmittel langsamer von statten geht. Ein Gehalt, der höher ist als die Obergrenze der angegebenen Bereiche führt zu höheren Kosten ohne wesentlichen Mehrnutzen:

Der Gesamtgewichtsanteil an dem wenigstens einen Halogenidsalz liegt im Bereich von 0,001 - 5 Gewichtsprozent, vorzugsweise 0,005 - 4 Gewichtsprozent und mehr bevorzugt 0,01 ― 4 Gewichtsprozent, bezogen auf das Gesamtgewicht der in den Kitkomponenten, vorzugsweise den Kitkomponenten (a), (b) und (c), enthaltenen Bestandteile. Ein Mindestgehalt an Halogenidsalzen von 0,00.1 Gewichtsprozent, bezogen auf das Gesamtgewicht der in den Kitkomponenten enthaltenen Bestandteilen, ist erforderlich, damit die Polymerisation in Gang gesetzt werden kann. Ein Gehalt von mehr als 5 Gewichtsprozent, bezogen auf das Gesamtgewicht der in den Kitkomponenten enthaltenen Bestandteile, erweist sich dagegen aufgrund der Toxizität der Halogenide als nachteilig.

Das Gesamtgewicht an dem wenigstens einen Vernetzer liegt im Bereich von 0,2 ― 3 Gewichtsprozent, vorzugsweise 0,5 ― 2,75 Gewichtsprozent und noch mehr bevorzugt 1 ― 2,5 Gewichtsprozent, bezogen auf das Gesamtgewicht der Bestandteile (a), (b) und (c). Der Anteil an Vernetzern in diesem Bereich ist essentiell, um dem ausgehärteten Knochenzement eine hohe Schlagzähigkeit zu verleihen. Es hat sich herausgestellt, dass sich bei einem Gehalt von unter 0,2 Gewichtsprozent und oberhalb von 3 Gewichtsprozent, jeweils bezogen auf das Gesamtgewicht der in den Kitkomponenten enthaltenen Bestandteile, die Schlagzähigkeit des ausgehärteten Knochenzementes signifikant verringert.

Gemäß einer bevorzugten Ausführungsform beträgt der Anteil der in Kitkomponente (a) enthaltenen Zusammensetzung 20 ― 40 Gewichtsprozent, der Anteil der in Kitkomponente (b) enthaltenen Zusammensetzung 20 ― 40 Gewichtsprozent und der Anteil der in Kitkomponente (c) enthaltenen Zusammensetzung 20 ― 40 Gewichtsprozent, bezogen auf das Gesamtgewicht der in den Kitkomponenten (a), (b) und (c) enthaltenen Verbindungen.

Erfindungsgemäß dient der Kit, der wenigstens die Kitkomponenten (a), (b) und (c) enthält, zur Herstellung von Knochenzement.

Dazu werden die wenigstens drei Kitkomponenten (a), (b) und (c) unter Ausbildung einer Paste miteinander vermischt.

Das Mischungsverhältnis beträgt vorzugsweise 0,5 ― 1,5 Gewichtsteile an Kitkomponente (a), 0,5 ― 1,5 Gewichtsteile an Kitkomponente (b) und 0,5 ― 1,5 Gewichtsteile an Kitkomponente (c).

Das Mischen kann mit üblichen Mischgeräten, beispielsweise einem statischen Mischer oder einem dynamischen Mischer erfolgen.

Nach dem Vermischen der Bestandteile des Kits ist die erhaltene Paste nach der Norm ISO 5833 klebfrei und kann sofort verarbeitet werden.

Die Paste kann beispielsweise zur Fixierung von Gelenkendoprothesen oder zum Auffüllen von Knochendefekten verwendet werden. Beide Verwendungen sind aus dem Stand der Technik in Verbindung mit herkömmlichen Pasten bekannt:

Der aus der Paste durch Aushärtung entstehende Knochenzement erreicht etwa sechs bis acht Minuten nach dem Mischen der einzelnen Kitkomponenten seine Endfestigkeit.

Die erfindungsgemäße Paste enthält wenigstens einen monofunktionellen, hydrophoben Methacrylsäureester (i).

Als monofunktioneller, hydrophober Methacrylsäureester (i) kommen alle hydrophoben Ester der Methacrylsäure in Betracht.

Durch die Verwendung von hydrophoben monofunktionellen Methacrylsäureestern (i) kann eine spätere Volumenvergrößerung des Knochenzements und damit eine Beschädigung des Knochens verhindert werden. Gemäß einer bevorzugten Ausführungsform ist der monofunktionelle Methacrylsäureester (i) hydrophob, wenn er neben der Estergruppe keine weiteren polaren Gruppen aufweist. Vorzugsweise weist der monofunktionelle, hydrophobe Methacrylsäureester (i) keine Carboxylgruppen, Hydroxylgruppen, Amidgruppen, Sulfonsäuregruppen, Sulfatgruppen, Phosphatgruppen oder Phosphonatgruppen auf.

Die erfindungsgemäß eingesetzten Methacrylsäureester (i) weisen vorzugsweise eine gewichtsmittlere Molmasse von weniger als 1000 g/mol auf.

Bei den Estern handelt es sich vorzugsweise um Alkylester. Die Alkylester sind gemäß einer bevorzugten Ausführungsform Ester von Methacrylsäure mit Alkoholen, die 1 ― 20 Kohlenstoffatome, mehr bevorzugt 1 ― 10 Kohlenstoffatome, noch mehr bevorzugt 1 ― 6 Kohlenstoffatome und ganz besonders bevorzugt 1 - 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem monofunktionellen, hydrophoben Methacrylsäureester (i) um Methacrylsäuremethylester oder Methacrylsäureethylester.

Bestandteil (i) enthält 15 ― 50 Gewichtsprozent, vorzugsweise 15 ― 45 Gewichtsprozent und noch mehr bevorzugt 20 ― 45 Gewichtsprozent wenigstens eines monofunktionellen, hydrophoben Methacrylsäureesters (i), bezogen auf das Gesamtgewicht der Paste. Demnach kann die Paste einen oder mehrere strukturell verschiedene monofunktionelle, hydrophobe Methacrylsäureester (i) enthalten, solange das Gesamtgewicht der monofunktionellen, hydrophoben Methacrylsäureester (i) im angegebenen Bereich liegt.

Die Paste enthält erfindungsgemäß einen Füllstoff (ii).

Bei dem in der Paste enthaltenen wenigstens einen Füllstoff (ii) handelt es sich um einen bei Raumtemperatur festen Stoff, der in der Lage ist, die Viskosität der Paste zu erhöhen. Der Füllstoff (ii) muss biokompatibel sein.

Gemäß einer bevorzugten Ausführungsform ist der Füllstoff (ii) aus der Gruppe ausgewählt, die aus (a) in wenigstens dem oder einem der Methacrylsäureester (i) löslichen Polymeren, (b) in wenigstens dem oder einem der Methacrylsäureester (i) unlöslichen Polymeren, (c) anorganischen Salzen, (d) anorganischen Oxiden, (e) Metallen und (f) Metalllegierungen besteht.

Bei dem in wenigstens einem der Methacrylsäureester (i) löslichen Polymer handelt es sich vorzugsweise um ein Polymer mit einer gewichtsmittleren Molmasse von wenigstens 150.000 g/mol. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAA), Polymethacrylsäurepropylester (PMAP), Polymethacrylsäureisopropylester und Polymethyl-co-acrylmethacrylat besteht.

Bei dem in wenigstens einem der Methacrylsäureester (i) unlöslichen Polymer handelt beispielsweise um Polyethylen, Polypropylen oder Polybutadien. Das in wenigstens einem der Methacrylsäureester (i) lösliche Polymer kann vernetzt oder nicht vernetzt sein.

Das als Füllstoff (ii) verwendbare anorganische Salz kann ein im Methacrylsäureester (i) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Calciumsulfat, Bariumsulfat oder Calciumcarbonat.

Bei dem als Füllstoff (ii) verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (ii) verwendbaren Metall kann es sich beispielsweise um ein Übergangsmetall handeln. Gemäß einer bevorzugten Ausführungsform ist das Metall Tantal oder Wolfram.

Die als Füllstoff (ii) verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln,

Der Anteil des wenigstens einen Füllstoffs (ii) beträgt 40 ― 85 Gewichtsprozent, vorzugsweise 40 ― 80 Gewichtsprozent und mehr bevorzugt 45 ― 75 Gewichtsprozent, bezogen auf das Gesamtgewicht der Paste. Dementsprechend kann die Paste einen oder mehrere strukturell verschiedene Füllstoffe (ii) enthalten, solange das Gesamtgewicht der Füllstoffe (ii) im angegebenen Bereich liegt.

Die Paste enthält ferner wenigstens einen in dem wenigstens einen monofunktionellen, hydrophoben Methacrylsäureester (i) löslichen Radikalinitiator (iii), der wenigstens eine Peroxidgruppe aufweist.

Unter Radikalinitiator (iii) wird vorliegend eine Verbindung verstanden, aus der durch Einwirkung des Akzelerators (v) ein Radikal gebildet werden kann, das in der Lage ist, die Polymerisation des wenigstens einen Methacrylsäureesters (i) auszulösen. Bei dem Radikalinitiator (iii) handelt es sich somit um einen radikalischen Polymerisationsstarter.

Der wenigstens eine Radikalinitiator (iii) weist in Gegenwart des Akzelerators (v) eine vom Radikalinitiator (iv) verschiedene Zerfallsgeschwindigkeit auf. Gemäß einer besonders bevorzugten Ausführungsform weist der der Radikalinitiator (iii) in Gegenwart des Akzelerators (v) eine niedrigere Zerfallsgeschwindigkeit auf als der Radikalinitiator (iv).

Gemäß einer bevorzugten Ausführungsform beträgt die Löslichkeit des wenigstens einen Radikalinitiators (iii) im wenigstens einen Methacrylsäureester (i) wenigstens 0,5 Gewichtsprozent, bezogen auf das Gewicht des wenigstens einen Methacrylsäureesters (i). Gemäß einer besonders bevorzugten Ausführungsform ist der Radikalinitiator (iii) aus der Gruppe ausgewählt, die aus Dibenzoylperoxid und Dilauroylperoxid besteht.

Die Paste enthält 0,01 ― 4 Gewichtsprozent, vorzugsweise 0,01 ― 3 Gewichtsprozent, mehr bevorzugt 0,05 - 2,5 Gewichtsprozent und noch mehr bevorzugt 0,05 ― 2 Gewichtsprozent wenigstens eines Radikalinitiators (iii), bezogen auf das Gesamtgewicht der Paste. Demnach kann die Paste einen oder mehrere strukturell verschiedene Radikalinitiatoren (iii) enthalten, solange das Gesamtgewicht der Radikalinitiatoren (iii) im angegebenen Bereich liegt.

Die Paste enthält zudem wenigstens einen in dem wenigstens einen monofunktionellen, hydrophoben Methacrylsäureester (i) löslichen Radikalinitiator (iv), der keine Peroxidgruppe aufweist.

Unter Radikalinitiator (iv) wird vorliegend eine Verbindung verstanden, aus der durch Einwirkung des Akzelerators (v) ein Radikal gebildet werden kann, das in der Lage ist, die Polymerisation des wenigstens einen Methacrylsäureesters (i) auszutösen. Bei dem Radikalinitiator (iv) handelt es sich somit um einen radikalischen Polymerisationsstarter.

Der wenigstens eine Radikalinitiator (iv) weist in Gegenwart des Akzelerators (v) eine vom Radikalinitiator (iii) verschiedene Zerfallsgeschwindigkeit auf. Gemäß einer besonders bevorzugten Ausführungsform weist der der Radikalinitiator (iv) in Gegenwart des Akzelerators (v) eine höhere Zerfallsgeschwindigkeit auf als der Rädikalinitiator (iii).

Gemäß einer bevorzugten Ausführungsform beträgt die Löslichkeit des wenigstens einen Radikalinitiators (iv) im wenigstens einen Methacrylsäureester (i) wenigstens 0,5 Gewichtsprozent, bezogen auf das Gewicht des wenigstens einen Methacrylsäureesters (i).

Bei dem Radikalinitiator (iv) handelt es sich vorzugsweise um Barbitursäure oder Barbitursäurederivate, bei denen die Barbitursäure an wenigstens einer der Positionen 1 oder 5 einen Substituenten trägt.

Das Barbitursäurederivat ist erfindungsgemäß vorzugsweise monosubstituiert. Gemäß einer bevorzugten Ausführungsform wird als Radikalinitiator (iv) eine an Position 5 substituierte Barbitursäure eingesetzt. Bei dem Substituenten des Barbitursäurederivats handelt es sich vorzugsweise ferner um einen hydrophoben Substituenten. Vorzugsweise handelt es sich bei dem Barbitursäurederivat um ein Alkyl-, Cycloalkyl- oder Arylderivat der Barbitursäure.

Gemäß einer besonders bevorzugten Ausführungsform ist das Barbitursäurederivat aus der Gruppe ausgewählt, die aus Cyclohexylbarbitursäure, 1,3,5-Trimethylbarbitursäure, 1-Phenyl-5-benzylbarbitursäure, 1-Benzyl-5-phenylbarbitursure, 1,3-Dimethylbarbitursäure, 1,3-Dimethyl-5-phenylbarbitursäure, 1-Cyclohexyl-5-ethylbarbitursäure, 5-Laurylbarbitursäure, 1-n-Butyl-barbitursäure, 5-n-Butylbarbitursäure, 5-Allylbarbitursäure, 5-Hydroxy-5-butylbarbitursäure, 5,5-Dibrombarbitursäure, Trichlorbarbitursäure, 5-Nitrobarbitursäure, 5-Aminobarbitursäure, 5-Hydroxybarbitursäure und 5,5-Dihydroxybarbitursäure besteht.

Unter einer ganz besonders bevorzugten Ausführungsform ist das Barbitursäurederivat aus der Gruppe ausgewählt, die aus 1-Cyclohexyl-5-ethyl-barbitursäure, 1-n-Butyl-barbitursäure und 5-n-Butyl-barbitursäure besteht.

Unter den Begriff Barbitursäurederivate fallen erfindungsgemäß auch Erdalkali- und Alkalisalze dieser Barbitursäurederivate.

Die Paste enthält 0,01 ― 4 Gewichtsprozent, vorzugsweise 0,01 ― 3 Gewichtsprozent, mehr be-vorzugt 0,05 - 2,5 Gewichtsprozent und noch mehr bevorzugt 0,05 ― 2 Gewichtsprozent wenigstens eines Radikalinitiators (iv), bezogen auf das Gesamtgewicht der Paste. Demnach kann die Paste einen oder mehrere strukturell verschiedene Radikalinitiatoren (iv) enthalten, solange das Gesamtgewicht des Radikalinitiators (iv) im angegebenen Bereich liegt.

Die Paste enthält ferner wenigstens einen in dem wenigstens einen Methacrylsäureester (i) löslichen Akzelerator (v), der in der Lage ist, aus den Radikalinitiatoren (iii) und (iv) Radikale zu bilden.

Gemäß einer bevorzugten Ausführungsform beträgt die Löslichkeit des Akzelerators (v) in dem wenigstens einen Methacrylsäureester (i) wenigstens 0,5 Gewichtsprozent, bezogen auf das Gewicht des wenigstens einen Methacrylsäureesters (i).

Unter Akzelerator, der in der Lage ist, aus den Radikalinitiatoren (iii) und (iv) Radikale zu bilden, werden Verbindungen verstanden, die die Radikalinitiatoren (iii) und (iv) gegebenenfalls in Gegenwart weiterer, in der erfindungsgemäßen Paste enthaltenen Verbindungen, wie beispielsweise Halogenidionen, zu Radikalen umsetzen können. Solche Akzeleratoren sind aus dem Stand der Technik gut bekannt.

Bei dem Akzelerator (v) handelt es sich vorzugsweise um ein Salz mit Ionen von Metallen, die neben der Oxidationsstufe 0 wenigstens zwei weitere Oxidationsstufen einnehmen können. Gemäß einer besonders bevorzugten Ausführungsform sind die Metallionen aus der Gruppe ausgewählt, die aus Kupferionen, Eisenionen, Cobaltionen und Manganionen besteht. Demnach wird als Akzelerator (v) vorzugsweise ein Eisensalz, ein Cobaltsalz oder ein Mangansalz eingesetzt. Gemäß einer ganz besonders bevorzugten Ausführungsform ist der Akzelerator (v) aus der Gruppe ausgewählt, die aus Kupfer(II)-2-ethylhexanoat, Kupfer(II)-laurat, Kupfer(II)-decanoat, Kupfer(II)-octoat, Kupfer(II)acetylacetonat und Kupfer(II)-methacrylat besteht.

Die Paste enthält 0,00001 ― 4 Gewichtsprozent, vorzugsweise 0,0001 ― 3 Gewichtsprozent, mehr bevorzugt 0,001 ― 3 Gewichtsprozent und noch mehr bevorzugt 0,05 ― 2 Gewichtsprozent wenigstens eines Akzelerators (v), bezogen auf das Gesamtgewicht der Paste. Demnach kann die Paste einen oder mehrere strukturell verschiedene Akzeleratoren (v) enthalten, solange das Gesamtgewicht des Akzelerators (v) im angegebenen Bereich liegt:

Die Paste enthält außerdem wenigstens ein Halogenidsalz (vi).

Bei dem Halogenidsalz (vi) handelt es sich vorzugsweise um ein in dem wenigstens einen Methacrylsäureester (i) lösliches Halogenidsalz.

Gemäß einer bevorzugten Ausführungsform beträgt die Löslichkeit des Halogenidsalzes (vi) in dem wenigstens einen Methacrylsäureester (i) wenigstens 0,5 Gewichtsprozent, bezogen auf das Gewicht des wenigstens einen Methacrylsäureesters (i).

Erfindungsgemäß werden als Halogenidsalze (vi). Salze verstanden, die wenigstens eine Art von Halogenidionen enthalten und in dem wenigstens einen Methacrylsäureester (i) dissoziieren. Vorzugsweise handelt es sich bei den Halogenidionen um Chloridionen oder Bromidionen, ganz bevorzugt um Chloridionen.

Erfindungsgemäß können als Halogenidsalze vorzugsweise Metallhalogenide, Hydrochloride und quarternäre Ammoniumhalogenidsalze eingesetzt werden.

Gemäß einer besonders bevorzugten Ausführungsform ist das Halogenidsalz (vi) aus der Gruppe ausgewählt, die aus Kupfer(II)*-*chlorid, Kupfer (II)-bromid, Eisen(III)-chlorid, Eisen(III)-bromid, Kobalt(II)-chlorid, Kobalt(II)-bromid, Triethylaminhydrochlorid, Triethylaminhydrobromid, Propylaminhydrochlorid, Butylaminhydrochlorid, Methacryloylcholinchlorid, Trioctylmethylammoniumchlorid und Triethylbenzylammoniumchlorid besteht.

Der Anteil des wenigstens einen Halogenidsalzes (vi) beträgt 0,001 ― 5 Gewichtsprozent, vorzugsweise 0,005 ― 4 Gewichtsprozent und mehr bevorzugt 0,01 ― 3 Gewichtsprozent, bezogen auf das Gesamtgewicht der Paste. Dementsprechend kann die Paste ein oder mehrere strukturell verschiedene Halogenidsalze (vi) enthalten, solange das Gesamtgewicht der Halogenidsalze (vi) im angegebenen Bereich liegt.

Die Paste enthält außerdem wenigstens einen Vernetzer (vii).

Unter Vernetzer wird vorliegend eine multifuriktionelle Verbindung verstanden, die in der Lage ist, mit wenigstens zwei voneinander verschiedenen Monomereinheiten kovalente Bindungen einzugehen.

Bei dem Vernetzer (vii) handelt es sich um eine bifunktionelle oder trifunktionelle Verbindung.

Erfindungsgemäß soll der Vernetzer eine Vernetzung des bei der Aushärtung des Knochenzementes polymerisierenden monofunktionellen, hydrophoben Methacrylsäureesters bewirken.

Gemäß einer bevorzugten Ausführungsform weist der Vernetzer (vii) wenigstens zwei Acrylatgruppen auf.

Besonders bevorzugt ist der Vernetzer (vii) aus der Gruppe ausgewählt, die aus Ethylenglycoldimethacrylat, Butylenglycoldimethacrylat (z.B. Butan-1 ,4-diol-dimethacrylat) und Hexamethylendimethacrylat (z.B. Hexan-1,6-diol-dimethacrylat) besteht.

Der Anteil des wenigstens einen Vernetzers (vii) beträgt 0,2 ― 3 Gewichtsprozent, vorzugsweise 0,5 ― 2,75 Gewichtsprozent und mehr bevorzugt 1 - 2,5 Gewichtsprozent, bezogen auf das Gesamtgewicht der Paste. Dementsprechend kann die Paste einen oder mehrere strukturell verschiedene Vernetzer (vii) enthalten, solange das Gesamtgewicht der Vernetzer (vii) im angegebenen Bereich liegt.

Zusätzlich kann die Paste noch weitere Bestandteile enthalten. Diese Bestandteile können beispielsweise aus der Gruppe ausgewählt sein, die aus pharmazeutisch aktiven Substanzen, Röntgenopakern, Farbstoffen und Stabilisatoren besteht.

Gemäß bevorzugter Ausführungsformen handelt es sich dabei um diejenigen pharmazeutisch aktiven Substanzen, Röntgenopaker, Farbstoffe und/oder Stabilisatoren, die vorstehend im Zusammenhang mit dem Kit beschrieben wurden.

Der erfindungsgemäße Kit oder die erfindungsgemäße Paste können vorzugsweise zur Fixierung von Gelenkendoprothesen verwendet werden.

Hierzu wird vorzugsweise aus dem erfindungsgemäßen Kit eine Paste hergestellt und diese analog zu den aus dem Stand der Technik bekannten Pasten zur Fixierung von Gelenkendoprothesen eingesetzt.

Ferner können der erfindungsgemäße Kit oder die erfindungsgemäße Paste auch zum Auffüllen von Knochendefekten verwendet werden.

Hierzu wird ebenfalls vorzugsweise aus dem erfindungsgemäßen Kit eine Paste hergestellt und diese analog zu den aus dem Stand der Technik bekannten Pasten zum Auffüllen von Knochendefekten eingesetzt.

### Ausführungsbeispiele:

Die in den Beispielen 1-3 eingesetzten Monomere und die weiteren Chemikalien lagen in pa.-Reinheit vor und wurden vom Chemikaliengroßhandel bezogen.

Es wurde Polymethylmethacrylat-co-methylacrylat mit einer Molmasse von ca. 200.000 g/mol verwendet. Dieses Polymer wird nachfolgend vereinfacht nur als PMMA bezeichnet Außerdem kam ein mit Ethylenglykoldimethacrylat vernetztes Polymethylmethacrylat zur Anwendung, das nachfolgend als vernetztes PMMA bezeichnet wird. Aliquat 336 steht nachfolgend für Trioctylmethylammoniumchlorid.

Die jeweiligen Pasten der Kitkomponenten (a), (b) und (c) wurden so hergestellt, dass zuerst in einem inerten Kunststoffgefäß das Methylmethacrylat eingewogen wurde. Danach wurden der Stabilisator und der jeweilige Initiator oder bei (c) der Akzelerator im Methylmethacrylat unter Rühren bei Raumtemperatur gelöst. Anschließend erfolgte die Zugabe aller weiteren Bestandteile. Die entstandenen Gemische wurden dann intensiv vermischt. Es bildeten sich streichfähige Pasten.

### Beispiel 1

| Kit 1 | | | | |
|---|---|---|---|---|
| Rezepturen | Methacrylsäure-ester | Füllstoffe | Radikalinitiator/Akzelerator | Stabilisator |
| Kitkomponente (a1) | 16,2 g Methylme-thacrylat; 0,6 g Ethylenglykol-dimethacrylat | 4,0 g Zirkonium-dioxid; 8,8 g PMMA; 9,2 g vernetztes PMMA; 0,4 g Methacrylamid | 0,8 g Dibenzoyl-peroxid (75 %ig) | 20 mg 2,6-Di-t-butyl-phenol |
| Kitkomponente (b1) | 16,2 g Methylme-thacrylat, 0,6 g Ethylenglykol-dimethacrylat | 4,0 g Zirkonium-dioxid; 8,8 g PMMA; 7,6 g vernetztes PMMA; 0,4 g Methacrylamid | 2,4 g 1-Cyclohexyl-5-ethyl-barbitursäure; 50 mg Aliquat 336 | 20 mg 2,6-Di-t-butyl-phenol |
| Kitkomponente (c1) | 16,2 g Methylme-thacrylat, 0,6 g Ethylenglykol-dimethacrylat | 4,6 g Zirkonium-dioxid; 8,6 g PMMA; 9,6 g vernetztes PMMA; 0,4 g Methacrylamid | 3 mg Kupfer(II)-2-ethyhexanoat | 20 mg 2,6-Di-t-butyl-phenol |

Die Kitkomponenten a1, b1 und c1 wurden intensiv miteinander vermischt. Es entstand ein streichbarer Zementteig, der sich bis zu ca. 4 Minuten verarbeiten ließ und nach ca. 6 Minuten ausgehärtet war. Der ausgehärtet Zement lag als weißer Festkörper vor.

### Beispiel 2

| Kit 2 | | | | |
|---|---|---|---|---|
| Rezepturen | Methacrylsäure-ester | Füllstoffe | Radikalinitiator/Akzelerator | Stabilisator |
| Kitkomponente (a2) | 16,2 g Methylme-thacrylat; 0,6 g Ethylenglykol-dimethacrylat | 4,0 g Zirkonium-dioxid; 8,8 g PMMA; 9,2 g vernetztes PMMA; 0,4 g Methacrylamid, | 0,8 g Dibenzoyl-peroxid (75 %ig) | 20 mg 2,6-Di-t-butyl-phenol |
| Kitkomponente (b2) | 16,2 g Methylme-thacrylat, 0,6 g Ethylenglykol-dimethacrylat | 4,0 g Zirkonium-dioxid; 8,8 g PMMA; 5,9 g vernetztes PMMA; 0,4 g Methacrylamid; 1,7 g Gentamicin-sulfat (AK 610) | 2,4 g 1-Cyclohexyl-5-ethyl-barbitursäure; | 20 mg 2,6-Di-t-butyl-phenol |
| Kitkomponente (c2) | 16,2 g Methylme-thacrylat, 0,6 g Ethylenglykol-dimethacrylat | 4,6 g Zirkonium-dioxid; 8,8 g PMMA; 9,4 g vernetztes PMMA; 0,4 g Methacrylamid | 3 mg Kupfer(II)-2-ethyhexanoat; 50 mg Aliquat 336, 10 mg 2-Ethylhexansäure | 20 mg 2,6-Di-t-butyl-phenol |

Die Kitkomponenten a2, b2 und c2 wurden intensiv miteinander vermischt. Es entstand ein streichbarer Zementteig, der sich bis zu ca. 4 Minuten verarbeiten ließ und nach ca. 6 Minuten ausgehärtet war. Der ausgehärtet Zement lag als weißer Festkörper vor.

Mit den Kits der Beispiele 1 und 2 wurden Zementprobekörper (Streifen) mit den Abmessungen 75 mm x 10 mm x 3,3 mm hergestellt. Für die Bestimmung der Biegefestigkeit und der Schlagzähigkeit (Dynstat-Methode) wurden Zementprobekörper der Abmessungen 16 mm x 10 mm x 3,3 mm gefertigt. Diese Probekörper wurden 24 Stunden bei 23 °C gelagert. Zusätzlich wurden Prüfkörper in Wasser für 24 Stunden bei 37 °C eingelagert.

Es wurde die 4-Punkt-Biegefestigkeit und das Biegemodul der gelagerten Probekörper mit Hilfe einer Zwick-Universalprüfmaschine ermittelt.

| Beispiele | Lagerungsbedingungen | 4-Punkt-Bieg | |
|---|---|---|---|
| | | Biegefestigkeit [MPa] | Biege-Modul [MPa] |
| Kit 1 | Luft/23°C/24 h | 58,2 ± 0,7 | 2374 ± 22 |
| Kit 1 | Wasser/37°C/24 h | 72,8 ± 1,6 | 2632 ± 28 |
| Kit 2 | Luft/23°C/24 h | 57,2 ± 0,8, | 2250 ± 48 |
| Kit 2 | Wasser/37°C/24 h | 59,8 ± 3,8 | 2286 ± 23 |

Die Bestimmung der Biegefestigkeit und der Schlagzähigkeit erfolgte mit einer Dynstat-Prüfapparatur.

| Beispiel | Lagerungsbedingungen | Biegefestigkeit [MPa] | Schlagzähigkeit [kJ/m²] |
|---|---|---|---|
| Kit 1 | Luft/23°C/24 h | 89,6 ± 1,8 | 4,91 ± 0,27 |
| Kit 1 | Wasser/37°C/24 h | 110,3 ± 2,7 | 4,49 ± 0,13 |
| Kit 2 | Luft/23°C/24 h | 76,8 ± 1,0 | 2,78 ± 0,08 |
| Kit 2 | Wasser/37°C/24 h | 72,5 ± 1,1 | 2,58 ± 0,27 |

### Beispiel 3

Die nachfolgend beschriebenen Kitkomponenten wurden analog zu den vorangegangenen Beispielen hergestellt.

### Kitkomponente (a)

| Rezepturen | Methacrylsäure-ester | Füllstoffe | Radikalinitiator | Stabilisator |
|---|---|---|---|---|
| A1 | 16,0 g Methylme-thacrylat, 0,4 g Ethylenglykol-dimethacrylat | 4,0 g Zirkonium-dioxid; 11,0 g PMMA; 7,7 g vernetztes PMMA; | 0,9 g Dibenzoylpe-roxid (75 %ig) | 20 mg 2,6-Di-t-butyl-phenol |
| A2 | 16,0 g Methylme-thacrylat, 0,4 g Butan-1,4-diol-dimethacrylat | 4,0 g Zirkonium-dioxid; 11,0 g PMMA; 7,7 g vernetztes PMMA; | 0,9 g Dibenzoylpe-roxid (75 %ig) | 20 mg 2,6-Di-t-butyl-phenol |
| A3 | 16,0 g Methylme-thacrylat, 0,4 g Hexan-1,6-dimethacrylat | 4,0 g Zirkonium-dioxid; 11,0 g PMMA; 6,2 g vernetztes PMMA; | 0,9 g Dibenzoylpe-roxid (75 %ig) | 20 mg 2,6-Di-t-butyl-phenol |
| A4 | 16,0 g Methylme-thacrylat, 0,4 g Ethylenglykol-dimethacrylat | 4,0 g Zirkonium-dioxid; 11,0 g PMMA; 6,2 g vernetztes PMMA; | 0,9 g Dibenzoylpe-roxid (75 %ig) | 40 mg 2,6-Di-t-butyl-phenol |
| A5 | 16,0 g Methylme-thacrylat, 0,4 g Butan-1,4-diol-dimethacrylat | 4,0 g Zirkonium-dioxid; 11,0 g PMMA; 6,2 g vernetztes PMMA; | 0,9 g Dibenzoylpe-roxid (75 %ig) | 40 mg 2,6-Di-t-butyl-phenol |
| A6 | 16,0 g Methylme-thacrylat, 0,4 g Hexan-1,6-dimethacrylat | 4,0 g Zirkonium-dioxid; 11,0 g PMMA; 6,2 g vernetztes PMMA; | 0,9 g Dibenzoylpe-roxid (75 %ig) | 60 mg 2,6-Di-t-butyl-phenol |
| A7 | 16,0 g Methylme-thacrylat, 0,4 g Ethylenglykol-dimethacrylat | 4,0 g Zirkonium-dioxid; 11,0 g PMMA; 7,8 g vernetztes PMMA; | 0,8 g Dilaurolype-roxid | 20 mg 2,6-Di-t-butyl-phenol |
| A8 | 14,0 g Methylme-thacrylat, 0,4 g Ethylenglykol-dimethacrylat | 4,0 g Zirkonium-dioxid; 11,0 g PMMA; 7,8 g vernetztes PMMA; | 0,8 g Dilaurolype-roxid | 20 mg 2,6-Di-t-butyl-phenol |
| A9 | 16,0 g Methylme-thacrylat, 0,4 g Hexan-1,6-dimethacrylat | 16,0 g Zirkonium-dioxid; 6,8 g PMMA; | 0,8 g Dilaurolype-roxid | 20 mg 2,6-Di-t-butyl-phenol |
| A10 | 16,0 g Methylme-thacrylat, 0,4 g Ethylenglykol-dimethacrylat | 4,0 g Bariumsulfat; 11,0g PMMA; 7,7 g vernetztes PMMA; | 0,9 g Dibenzoylpe-roxid | 20 mg 2,6-Di-t-butyl-phenol |
| A11 | 16,0 g Methylme-thacrylat, 0,4 g Ethylenglykol-dimethacrylat | 6,0 g Calciumcar-bonat; 11,0 g PMMA; 5,7 g vernetztes PMMA | 0,9 g Dibenzoylpe-roxid | 20 mg 2,6-Di-t-butyl-phenol |
| A12 | 16,0 g Methylme-thacrylat, 0,4 g Hexan-1,6-dimethacrylat | 4,0 g Tantalpulver; 11,0 g PMMA; 7,7 g vernetztes PMMA; | 0,9 g Dibenzoylpe-roxid | 20 mg 2,6-Di-t-butyl-phenol |

### Kitkomponenten (b)

| Rezepturen | Methacrylsäure-ester | Füllstoffe | Radikalinitiator | Stabilisator |
|---|---|---|---|---|
| B1 | 16,0 g Methylme-thacrylat, 0,4 g Ethylenglykol-dimethacrylat | 4,0 g Zirkonium-dioxid; 11,0 g PMMA; 6,2 g vernetztes PMMA; | 2,4 g 1-Cyclohexyl-5-ethylbarbitursäure; 60 mg Aliquat 336 | 20 mg 2,6-Di-t-butyl-phenol |
| B2 | 16,0 g Methylme-thacrylat, 0,4 g Butan-1,4-diol-dimethacrylat | 4,0 g Zirkonium-dioxid; 11,0 g PMMA; 6,2 g vernetztes PMMA; | 2,4 g 1-Cyclohexyl-5-ethylbarbitursäure; 60 mg Aliquat 336 | 20 mg 2,6-Di-t-butyl-phenol |
| B3 | 16,0 g Methylme-thacrylat, 0,4 g Hexan-1,6-dimethacrylat | 4,0 g Zirkonium-dioxid; 11,0 g PMMA; 6,2 g vernetztes PMMA; | 2,4 g 1-Cyclohexyl-5-ethylbarbitursäure; 60 mg Aliquat 336 | 20 mg 2,6-Di-t-butyl-phenol |
| B4 | 16,0 g Methylme-thacrylat, 0,4 g Ethylenglykol-dimethacrylat | 4,0 g Zirkonium-dioxid; 11,0 g PMMA; 6,2 g vernetztes PMMA; | 2,4 g 1-Cyclohexyl-5-ethylbarbitursäure; 60 mg Aliquat 336 | 40 mg 2,6-Di-t-butyl-phenol |
| B5 | 16,0 g Methylme-thacrylat, 0,4 g Butan-1,4-diol-dimethacrylat | 4,0 g Zirkonium-dioxid; 11,0 g PMMA; 6,2 g vernetztes PMMA; | 2,4 g 1-Cyclohexyl-5-ethylbarbitursäure; 60 mg Aliquat 336 | 40 mg 2,6-Di-t-butyl-phenol |
| B6 | 16,0 g Methylme-thacrylat, 0,4 g Hexan-1,6-dimethacrylat | 4,0 g Zirkonium-dioxid; 11,0 g PMMA; 6,2 g vernetztes PMMA; | 2,4 g 1-Cyclohexyl-5-ethylbarbitursäure; 60 mg Aliquat 336 | 60 mg 2,6-Di-t-butyl-phenol |
| B7 | 16,0 g Methylme-thacrylat, 0,4 g Ethylenglykol-dimethacrylat | 4,0 g Zirkonium-dioxid; 11,0 g PMMA; 6,2 g vernetztes PMMA; | 2,4 g 1-n-Butyl-5-n-butyl-barbitursäure; 60 mg Aliquat 336 | 20 mg 2,6-Di-t-butyl-phenol |
| B8 | 14,0 g Methylme-thacrylat, 0,4 g Ethylenglykol-dimethacrylat | 4,0 g Zirkonium-dioxid; 11,0 g PMMA; 8,2 g vernetztes PMMA; | 2,4 g 1-Cyclohexyl-5-ethylbarbitursäure; 60 mg Aliquat 336 | 20 mg 2,6-Di-t-butyl-phenol |
| B9 | 16,0 g Methylme-thacrylat, 0,4 g Hexan-1,6-dimethacrylat | 16,0 g Zirkonium-dioxid; 5,2 g PMMA; | 2,4 g 1-Cyclohexyl-5-ethylbarbitursäure; 60 mg Aliquat 336 | 20 mg 2,6-Di-t-butyl-phenol |
| B10 | 16,0 g Methylme-thacrylat, 0,4 g Ethylenglykol-dimethacrylat | 4,0 g Bariumsulfat; 11,0 g PMMA; 6,2 g vernetztes PMMA; | 2,4 g 1-n-Butyl-5-n-butyl-barbitursäure; 60 mg Aliquat 336 | 20 mg 2,6-Di-t-butyl-phenol |
| B11 | 16,0 g Methylme-thacrylat, 0,4 g Ethylenglykol-dimethacrylat | 6,0 g Calciumcar-bonat; 11,0 g PMMA; 4,2 g vernetztes PMMA; | 2,4 g 1-n-butyl-5-n-butyl-barbitursäure; 60 mg Aliquat 336 | 20 mg 2,6-Di-t-butyl-phenol |
| B12 | 16,0 g Methylme-thacrylat, 0,4 g Hexan-1,6-dimethacrylat | 4,0 g Tantalpulver; 11,0 g PMMA; 6,2 g vernetztes PMMA; | 2,4 g 1-Cyclohexyl-5-ethylbarbitursäure; 60 mg Aliquat 336 | 60 mg 2,6-Di-t-butyl-phenol |
| B13 | 16,0 g Methylme-thacrylat, 0,4 g Ethylenglykol-dimethacrylat | 4,0 g Zirkonium-dioxid; 11,0 g PMMA; 4,6 g vernetztes PMMA; 1,6 g Gentamicin-sulfat | 2,4 g 1-Cyclohexyl-5-ethylbarbitursäure; 60 mg Aliquat 336 | 20 mg 2,6-Di-t-butyl-phenol |
| B14 | 14,0 g Methylme-thacrylat, 0,4 g Ethylenglykol-dimethacrylat | 4,0 g Zirkonium-dioxid; 11,0 g PMMA; 8,2 g vernetztes PMMA; 5 mg Lissaming-rün, 5 mg 2-Ethyl-hexansäure | 2,4 g 1-Cyclohexyl-5-ethylbarbitursäure; 60 mg Aliquat 336 | 20 mg 2,6-Di-t-butyl-phenol |
| B15 | 14,0 g Methylme-thacrylat, 0,4 g Ethylenglykol-dimethacrylat | 4,0 g Zirkonium-dioxid; 11,0 g PMMA; 8,2 g vernetztes PMMA; 5 mg Lissaming-rün, 5 mg 2-Ethyl-hexansäure | 2,4 g 1-Cyclohexyl-5-ethylbarbitursäure; 60 mg Tetrabuty-lammoniumchlorid | 20 mg 2,6-Di-t-butyl-phenol |

### Kitkomponenten (c)

| Rezepturen | Methacrylsäure-ester | Füllstoffe | Akzelerator | Stabilisator |
|---|---|---|---|---|
| C1 | 16,0 g Methylme-thacrylat, 0,4 g Ethylenglykol-dimethacrylat | 4,0 g Zirkonium-dioxid; 11,0 g PMMA; 8,6 g vernetztes PMMA; | 3 mg Kupfer(II)-2-ethylhexanoat | 20 mg 2,6-Di-t-butyl-phenol |
| C2 | 16,0 g Methylme-thacrylat, 0,4 g Butan-1,4-diol-dimethacrylat | 4,0 g Zirkonium-dioxid; 11,0 g PMMA; 8,6 g vernetztes PMMA; | 3 mg Kupfer(II)-2-ethylhexanoat | 20 mg 2,6-Di-t-butyl-phenol |
| C3 | 16,0 g Methylme-thacrylat, 0,4 g Hexan-1,6-dimethacrylat | 4,0 g Zirkonium-dioxid; 11,0 g PMMA; 8,6 g vernetztes PMMA; | 3 mg Kupfer(II)-2-ethylhexanoat | 20 mg 2,6-Di-t-butyl-phenol |
| C4 | 16,0 g Methylme-thacrylat, 0,4 g Ethylenglykol-dimethacrylat | 4,0 g Zirkonium-dioxid; 11,0 g PMMA; 8,6 g vernetztes PMMA; | 2 mg Kupfer(II)-methacrylat | 40 mg 2,6-Di-t-butyl-phenol |
| C5 | 16,0 g Methylme-thacrylat, 0,4 g Butan-1,4-diol-dimethacrylat | 4,0 g Zirkonium-dioxid; 11,0 g PMMA; 8,6 g vernetztes PMMA; | 2 mg Kupfer(II)-methacrylat | 40 mg 2,6-Di-t-butyl-phenol |
| C6 | 16,0 g Methylme-thacrylat, 0,4 g Hexan-1,6-dimethacrylat | 4,0 g Zirkonium-dioxid; 11,0 g PMMA; 8,6 g vernetztes PMMA; | 2 mg Kupfer(II)-methacrylat | 60 mg 2,6-Di-t-butyl-phenol |
| C7 | 16,0 g Methylme-thacrylat, 0,4 g Ethylenglykol-dimethacrylat | 4,0 g Zirkonium-dioxid; 11,0 g PMMA; 8,6 g vernetztes PMMA; | 2 mg Kupfer(II)-acetylacetonat | 20 mg 2,6-Di-t-butyl-phenol |
| C8 | 14,0 g Methylme-thacrylat, 0,4 g Ethylenglykol-dimethacrylat | 6,0 g Zirkonium-dioxid; 11,0 g PMMA; 8,6 g vernetztes PMMA; | 2 mg Kupfer(II)-acetylacetonat | 20 mg 2,6-Di-t-butyl-phenol |
| C9 | 16,0 g Methylme-thacrylat, 0,4 g Hexan-1,6-dimethacrylat | 16,0 g Zirkonium-dioxid; 7,6 g PMMA; | 2 mg Kupfer(II)-acetylacetonat | 20 mg 2,6-Di-t-butyl-phenol |
| C10 | 16,0 g Methylme-thacrylat, 0,4 g Ethylenglykol-dimethacrylat | 4,0 g Bariumsulfat; 11,0 g PMMA; 8,6 g vernetztes PMMA; | 3 mg Kupfer(II)-2-ethylhexanoat | 20 mg 2,6-Di-t-butyl-phenol |
| C11 | 16,0 g Methylme-thacrylat, 0,4 g Ethylenglykol-dimethacrylat | 6,0 g Calciumcar-bonat; 11,0 g PMMA; 6,6 g vernetztes PMMA | 3 mg Kupfer(II)-2-ethylhexanoat | 20 mg 2,6-Di-t-butyl-phenol |
| C12 | 16,0 g Methylme-thacrylat, 0,4 g Hexan-1,6-dimethacrylat | 4,0 g Tantalpulver; 11,0 g PMMA; 8,6 g vernetztes PMMA; | 3 mg Kupfer(II)-2-ethylhexanoat | 20 mg 2,6-Di-t-butyl-phenol |

## Patentansprüche

1. Kit umfassend
eine Kitkompohente (a), die als Bestandteile wenigstens (a1) einen monofunktionellen, hydrophoben Methacrylsäureester, (a2) einen Füllstoff und (a3) einen in (a1) löslichen Radikalinitiator, der wenigstens eine Peroxidgruppe aufweist, enthält, wobei Kitkomponente (a) 15 - 85 Gewichtsprozent Methacrylsäureester (a1) und weniger als 85 Gewichtsprozent Füllstoffe (a2), bezogen auf das Gesamtgewicht der in Kitkomponente (a) enthaltenen Bestandteile aufweist,
eine Kitkomponente (b), die als Bestandteile wenigstens (b1) einen monofunktionellen, hydrophoben Methacrylsäureester, (b2) einen Füllstoff und (b3) einen in (b1) löslichen Radikalinitiator, der keine Peroxidgruppe aufweist, enthält, wobei Kitkomponente (b) 15 - 85 Gewichtsprozent Methacrylsäureester (b1) und weniger als 85 Gewichtsprozent Füllstoffe (b2), bezogen auf das Gesamtgewicht der in Kitkomponente (b) enthaltenen Bestandteile aufweist,
eine Kitkompönente (c), die als Bestandteile wenigstens (c1) einen monofunktionellen, hydrophoben Methacrylsäureester, (c2) einen Füllstoff und (c3) einen in (c1) löslichen Akzelerator, der in der Lage ist, aus den Radikalinitiatoren (a3) und (b3) Radikale zu bilden, enthält, wobei Kitkomponente (c) 15 - 85 Gewichtsprozent Methacrylsäureester (c1) und weniger als 85 Gewichtsprozent Füllstoffe (c2), bezogen auf das Gesamtgewicht der in Kitkomponente (c) enthaltenen Bestandteile aufweist,
wobei wenigstens eine der Kitkomponenten (a), (b) oder (c) wenigstens ein Halogenidsalz enthält und
wenigstens eine der Kitkomponenten (a), (b) oder (c) wenigstens einen Vernetzer enthält,
wobei, bezogen auf das Gesamtgewicht der in den Kitkomponenten (a), (b) und (c) enthaltenen Bestandteile, (i) der Gesamtgewichtsanteil an den Methacrylsäureestern (a1), (b1) und (c1) im Bereich von 15 - 50 Gewichtsprozent, (ii) der Gesamtgewichtsanteil an den Füllstoffen (a2), (b2) und (c2) im Bereich von 40 - 85 Gewichtsprozent, (iii) der Gewichtsanteil am Radikalinitiator (a3) im Bereich von 0,01 - 4 Gewichtsprozent, (iv) der Gewichtsanteil am Radikalinitiator (b3) im Bereich von 0,01 -4 Gewichtsprozent, (v) der Gewichtsanteil am Akzelerator (c3) im Bereich von 0,000001 - 3 Gewichtsprozent, (vi) der Gesamtgewichtsanteil an dem wenigstens einen Halogenidsalz im Bereich von 0,001 - 5 Gewichtsprozent und (vii) der Gesamtgewichtsanteil an dem wenigstens einen Vernetzer im Bereich von 0,2 - 3 Gewichtsprozent liegt.

2. Kit nach Anspruch 1, **dadurch gekennzeichnet, dass** wenigstens einer der Methacrylsäureester (a1), (b1) und (c1) aus der Gruppe ausgewählt ist, die aus Methacrylsäuremethylester und Methacrylsäureethylester besteht.

3. Kit nach Anspruch 1 oder 2, **dadurch gekennzeichnet, dass** weinigstens einer der Füllstoffe (a2), (b2) und (c2) aus der Gruppe ausgewählt ist, die aus in wenigstens einem der Methacrylsäureester (a1), (b1) und (c1) löslichen Polymeren, in wenigstens einem der Methacrylsäureester (a1), (b1) und (c1) unlöslichen Polymeren, anorganischen Salzen, anorganischen Oxiden, Metallen und Metalllegierungen besteht.

4. Kit nach Anspruch 3, **dadurch gekennzeichnet, dass** das in wenigstens einem der Methacrylsäureester (a1), (b1) und (c1) lösliche Polymer eine gewichtsmittlere Molmasse von wenigstens 150.000 g/mol aufweist.

5. Kit nach einem der Ansprüche 1 bis 4, **dadurch gekennzeichnet, dass** der Radikalinitiator (a3) aus der Gruppe ausgewählt ist, die aus Dibenzoylperoxid und Dilauroylperoxid besteht.

6. Kit nach einem der Ansprüche 1 bis 5, **dadurch gekennzeichnet, dass** der Radikalinitiator (b3) aus der Gruppe ausgewählt ist, die aus Barbitursäure und an Position 1 oder 5 substituierten Barbitursäuren besteht.

7. Kit nach Anspruch 6, **dadurch gekennzeichnet, dass** der Radikalinitiator (b3) aus der Gruppe ausgewählt ist, die aus 1-Cyclohexyl-5-ethyl-barbitursäure, 1-n-Butyl-barbitursäure und 5-Butyl-barbitursäure besteht.

8. Kit nach einem der Ansprüche 1 bis 7, **dadurch gekennzeichnet, dass** der Akzelerator (c3) aus der Gruppe ausgewählt ist, die aus Salzen mit Ionen von Metallen besteht, die neben der Oxidationsstufe 0 wenigstens zwei weitere Oxidationsstufen einnehmen können.

9. Kit nach Anspruch 8, **dadurch gekennzeichnet, dass** die Metallionen aus der Gruppe ausgewählt sind, die aus Kupferionen, Eisenionen, Cobaltionen und Manganionen besteht.

10. Kit nach Anspruch 8 oder 9, **dadurch gekennzeichnet, dass** der Akzelerator (c3) aus der Gruppe ausgewählt ist, die aus Kupfer(II)-2-ethylhexanoat, Kupfer(II)-laurat, Kupfer(II)-decanoat, Kupfer(II)-octoat, Kupfer(II)acetylacetonat und Kupfer(II)-methacrylat besteht.

11. Kit nach einem der Ansprüche 1 bis 10, **dadurch gekennzeichnet, dass** das wenigstens eine Halogenidsalz aus der Gruppe ausgewählt ist, die aus Metallhalogeniden, Hydrochloriden und quarternären Ammoniumhalogenidsalzen besteht.

12. Kit nach einem der Ansprüche 1 - 11, **dadurch gekennzeichnet, dass** der wenigstens eine Vernetzer aus der Gruppe ausgewählt ist, die aus multifunktionellen Methacrylatmonomeren besteht.

13. Kit nach Anspruch 12, **dadurch gekennzeichnet, dass** das multifunktionelle Methacrylatmonomer aus der Gruppe ausgewählt ist, die aus Ethylenglycoldimethacrylat, Butylenglycoldimethacrylat und Hexamethylendimethacrylat besteht.

14. Kit nach einem der Ansprüche 1 -13, **dadurch gekennzeichnet, dass** wenigstens eine der Kitkomponenten (a), (b) und (c) wenigstens eine pharmazeutisch aktive Substanz enthält.

15. Kit nach Anspruch 14, **dadurch gekennzeichnet, dass** die wenigstens eine pharmazeutisch aktive Substanz aus der Gruppe ausgewählt ist, die aus Antibiotika, Antiphlogistika, Hormonen, Wachstumsfaktoren, Bisphosphönaten und Cytostatika besteht.

16. Kit nach einem der Ansprüche 1-15, **dadurch gekennzeichnet, dass** wenigstens eine der Kitkomponenten (a), (b) und (c) wenigstens einen Röntgenopaker enthält.

17. Kit nach einem der Ansprüche 1 -16, **dadurch gekennzeichnet, dass** wenigstens eine der Kitkomponenten (a), (b) und (c) einen Farbstoff enthält.

18. Paste umfassend
(i) 15 - 50 Gewichtsprozent wenigstens eines monofunktionellen, hydrophoben Methacrylsäureesters,
(ii) 40 - 85 Gewichtsprozent wenigstens eines Füllstoffs,
(iii) 0,01 - 4 Gewichtsprozent wenigstens eines im Methacrylsäureester (i) löslichen Radikalinitiators, der wenigstens eine Peroxidgruppe aufweist,
(iv) 0,01 -4 Gewichtsprozent wenigstens eines im Methacrylsäureester (i) löslichen Radikalinitiators, der keine Peroxidgruppe aufweist,
(v) 0,000001 - 3 Gewichtsprozent wenigstens eines im Methacrylsäureester (i) löslichen Akzelerators, der in der Lage ist, aus den Radikalinitiatoren gemäß (iii) und (iv) Radikale zu bilden,
(vi) 0,001 - 5 Gewichtsprozent wenigstens eines Halogenidsalzes und
(vii) 0,2 - 3 Gewichtsprozent wenigstens eines Vernetzers.

19. Verwendung eines Kits nach einem der Ansprüche 1 - 17 oder einer Paste nach Anspruch 18 zur Fixierung von Gelenkendoprothesen: .

20. Verwendung eines Kits nach einem der Ansprüche 1 -17 oder einer Paste nach Anspruch 18 zum Auffüllen von Knochendefekten.

## Claims

1. Kit, comprising
a kit component (a) containing, as ingredients, at least (a1) one monofunctional hydrophobic methacrylic acid ester, (a2) a filling agent, and (a3) a radical initiator that is soluble in (a1) and comprises at least one peroxide group, whereby kit component (a) comprises 15 - 85 % by weight methacrylic acid ester (a1) and less than 85 % by weight filling agents (a2), with respect to the total weight of the ingredients contained in kit component (a);
a kit component (b) containing, as ingredients, at least (b1) one monofunctional hydrophobic methacrylic acid ester, (b2) a filling agent, and (b3) a radical initiator that is soluble in (b1) and comprises no peroxide group, whereby kit component (b) comprises 15 - 85 % by weight methacrylic acid ester (b1) and less than 85 % by weight filling agents (b2), with respect to the total weight of the ingredients contained in kit component (b);
a kit component (c) containing, as ingredients, at least (c1) one monofunctional hydrophobic methacrylic acid ester, (c2) a filling agent, and (c3) an accelerator that is soluble in (c1) and is capable of forming radicals from radical initiators (a3) and (b3), whereby kit component (c) comprises 15 - 85 % by weight methacrylic acid ester (c1) and less than 85 % by weight filling agents (c2), with respect to the total weight of the ingredients contained in kit component (c);
whereby at least one of the kit components (a), (b) or (c) contains at least on halogenide salt; and
at least one of the kit components (a), (b) or (c) contains at least one cross-linker;
whereby, with respect to the total weight of the ingredients contained in the kit components (a), (b) or (c), (i) the total weight fraction of the methylacrylic acid esters (a1), (b1) and (c1) is in the range of 15 - 50 % by weight, (ii) the total weight fraction of the the filling agents (a2), (b2), and (c2) is in the range of 40 - 85 % by weight, (iii) the weight fraction of the radical initiator (a3) is in the range of 0.01 - 4 % by weight, (iv) the weight fraction of the radical initiator (b3) is in the range of 0.01 - 4 % by weight, (v) the weight fraction of the accelerator (c3) is in the range of 0.000001 - 3 % by weight, (vi) the total weight fraction of the at least one halogenide salt is in the range of 0.001 - 5 % by weight, and (vii) the total weight fraction of the at least one cross-linker is in the range of 0.2 - 3 % by weight.

2. Kit according to claim 1, **characterised in that** at least one of the methacrylic acid esters (a1), (b1) and (c1) is selected from the group consisting of methacrylic acid methyl ester and methacrylic acid ethyl ester.

3. Kit according to claim 1 or 2, **characterised in that** at least one of the filling agents (a2), (b2) and (c2) is selected from the group consisting of polymers that are soluble in at least one of the methacrylic acid esters (a1), (b1) and (c1), polymers that are insoluble in at least one of the methacrylic acid esters (a1), (b1) and (c1), inorganic salts, inorganic oxides, metals, and metal alloys.

4. Kit according to claim 3, **characterised in that** the polymer that is soluble in at least one of the methacrylic acid esters (a1), (b1) and (c1) has a weight-averaged molar mass of at least 150,000 g/mol.

5. Kit according to any one of the claims 1 to 4, **characterised in that** the radical initiator (a3) is selected from the group consisting of dibenzoylperoxide and dilauroylperoxide.

6. Kit according to any one of the claims 1 to 5, **characterised in that** the radical initiator (b3) is selected from the group consisting of barbituric acid and barbituric acids substituted at position 1 or 5.

7. Kit according to claim 6, **characterised in that** the radical initiator (b3) is selected from the group consisting of 1-cyclohexyl-5-ethyl-barbituric acid, 1-n-butyl-barbituric acid, and 5-butyl-barbituric acid.

8. Kit according to any one of the claims 1 to 7, **characterised in that** the accelerator (c3) is selected from the group consisting of salts including ions of metals which can take two more oxidation stages in addition to oxidation stage 0.

9. Kit according to claim 8, **characterised in that** the metal ions are selected from the group consisting of copper ions, iron ions, cobalt ions, and manganese ions.

10. Kit according to claim 8 or 9, **characterised in that** the accelerator (c3) is selected from the group consisting of copper(II)-2-ethylhexanoate, copper(II)-laurate, copper(II)-decanoate, copper(II)-octoate, copper(II)-acetylacetonate, and copper(II)-methacrylate.

11. Kit according to any one of the claims 1 to 10, **characterised in that** the at least one halogenide salt is selected from the group consisting of metal halogenides, hydrochlorides, and quarternary ammonium halogenide salts.

12. Kit according to any one of the claims 1 - 11, **characterised in that** the at least one cross-linker is selected from the group consisting of multifunctional methacrylate monomers.

13. Kit according to claim 12, **characterised in that** the multifunctional methacrylate monomer is selected from the group consisting of ethylene glycol dimethacrylate, butylene glycol dimethacrylate, and hexamethylene dimethacrylate.

14. Kit according to any one of the claims 1 - 13, **characterised in that** at least one of the kit components (a), (b), and (c) contains at least one pharmaceutically active substance.

15. Kit according to claim 14, **characterised in that** the at least one pharmaceutically active substance is selected from the group consisting of antibiotics, antiphlogistics, hormones, growth factors, bisphosphonates, and cytostatic agents.

16. Kit according to any one of the claims 1 - 15, **characterised in that** at least one of the kit components (a), (b), and (c) contains at least one radiopaquer.

17. Kit according to any one of the claims 1 - 16, **characterised in that** at least one of the kit components (a), (b), and (c) contains a dye.

18. Paste, comprising
(i) 15 - 50 % by weight of at least one monofunctional hydrophobic methacrylic acid ester;
(ii) 40 - 85 % by weight of at least one filling agent;
(iii) 0.01 - 4 % by weight of at least one radical initiator that is soluble in methacrylic ester (i) and comprises at least one peroxide group;
(iv) 0.01 - 4 % by weight of at least one radical initiator that is soluble in methacrylic ester (i) and comprises no peroxide group;
(v) 0.000001 - 3 % by weight of at least one accelerator that is soluble in methacrylic acid ester (i) and capable of forming radicals from the radical initiators according to (iii) and (iv);
(vi) 0.001 - 5 % by weight of at least one halogenide salt; and
(vii) 0.2 - 3 % by weight of at least one cross-linker.

19. Use of a kit according to any one of the claims 1 - 17 or of a paste according to claim 18 for affixing articular endoprostheses.

20. Use of a kit according to any one of the claims 1 - 17 or of a paste according to claim 18 for filling up bone defects.

## Revendications

1. Kit comprenant
un composante de kit (a) qui contient en tant que composant, au moins (a1) un ester d'acide méthacrylique hydrophobe monofonctionnel, (a2) une charge et (a3) un initiateur de radical soluble dans (a1) qui présente au moins un groupe peroxyde, sachant que la composante (a) du kit présente 15 - 85 pourcents en poids d'esters d'acide méthacrylique (a1) et moins de 85 pourcents en poids de charges (a2), rapporté au poids total des composants contenus dans la composante de kit (a),
une composante de kit (b) qui contient en tant que composant, au moins (b1) un ester d'acide méthacrylique hydrophobe monofonctionnel, (b2) une charge et (b3) un initiateur de radical soluble dans (b1) qui ne présente pas de groupe peroxyde, sachant que la composante (b) du kit présente 15 - 85 pourcents en poids d'esters d'acide méthacrylique (b1) et moins de 85 pourcents en poids de charges (b2), rapporté au poids total des composants contenus dans la composante de kit (b),
une composante de kit (c), qui contient en tant que composant, au moins (c1) un ester d'acide méthacrylique hydrophobe monofonctionnel, (c2) une charge et (c3) un accélérateur soluble dans (c1) qui est capable de former des radicaux à partir des initiateurs de radical (a3) et (b3), sachant que la composante (c) du kit présente 15 - 85 pourcents en poids d'esters d'acide méthacrylique (c1) et moins de 85 pourcents en poids de charges (c2), rapporté au poids total des composants contenus dans la composante de kit (c),
sachant qu'au moins une des composantes (a), (b) ou (c) du kit contient au moins un sel d'halogénure et
au moins une des composantes (a), (b) ou (c) du kit contient au moins un réticulant,
sachant que, rapporté au poids total des composants contenus dans les composantes (a), (b) ou (c) du kit, (i) la teneur en poids totale d'esters d'acide méthacrylique (a1), (b1) et (c1) est de l'ordre de 15 - 50 pourcents en poids, (ii) la teneur en poids totale des charges (a2), (b2) et (c2) est de l'ordre de 40 - 85 pourcents en poids, (iii) la teneur en poids totale d'initiateur de radical (a3) est de l'ordre de 0,01 - 4 pourcents en poids, (iv) la teneur en poids totale d'initiateur de radical (b3) est de l'ordre de 0,01 - 4 pourcents en poids, (v) la teneur en poids totale d'accélérateur (c3) est de l'ordre de 0,000001 - 3 pourcents en poids, (vi) la teneur en poids totale de l'au moins un sel d'halogénure est de l'ordre de 0,001 - 5 pourcents en poids et (vii) la teneur en poids totale de l'au moins un réticulant est de l'ordre de 0,2 - 3 pourcents en poids.

2. Kit selon la revendication 1, **caractérisé en ce qu'**au moins un des esters d'acide méthacrylique (a1), (b1) et (c1) est choisi dans le groupe constitué d'esters d'acide méthacrylique et d'esters éthyliques d'acide méthacrylique.

3. Kit selon la revendication 1 ou 2, **caractérisé en ce qu'**au moins une des charges (a2), (b2) et (c2) est choisie dans le groupe constitué de polymères solubles dans au moins un des esters d'acide méthacrylique (a1), (b1) et (c1), de polymères non solubles dans au moins un des esters d'acide méthacrylique (a1), (b1) et (c1), de sels anorganiques, d'oxydes anorganiques, de métaux et d'alliages métalliques.

4. Kit selon la revendication 3, **caractérisé en ce que** le polymère soluble dans au moins un des esters d'acide méthacrylique (a1), (b1) et (c1) présente une masse molaire moyenne en poids d'au moins 150 000 g/mol.

5. Kit selon l'une des revendications 1 à 4, **caractérisé en ce que** l'initiateur de radical (a3) est sélectionné parmi le groupe constitué de peroxyde de dibenzoyle et de peroxyde dilauroyle.

6. Kit selon l'une des revendications 1 à 5, **caractérisé en ce que** l'initiateur de radical (b3) est sélectionné parmi le groupe constitué d'acide barbiturique et d'acides barbituriques substitués en position 1 ou 5.

7. Kit selon la revendication 6, **caractérisé en ce que** l'initiateur de radical (b3) est sélectionné parmi le groupe constitué d'acide barbiturique 1-cyclohexylique-5-éthylique, d'acide barbiturique 1-n-butyle et d'acide barbiturique 5-butyle.

8. Kit selon l'une des revendications 1 à 7, **caractérisé en ce que** l'accélérateur (c3) est sélectionné parmi le groupe constitué de sels avec des ions métalliques qui peuvent prendre au moins deux autres degrés d'oxydation en plus du degré d'oxydation 0.

9. Kit selon la revendication 8, **caractérisé en ce que** les ions métalliques sont sélectionnés parmi le groupe constitué d'ions cuivre, d'ions fer, d'ions cobaltet d'ions manganèse.

10. Kit selon la revendication 8 ou 9, **caractérisé en ce que** l'accélérateur (c3) est sélectionné parmi le groupe constitué du 2-éthylhexanoate de cuivre(Il), du laurate de cuivre(II), du décanoate de cuivre(II), de l'octoate de cuivre(II), de l'acétylacétonate de cuivre(II) et du méthacrylate de cuivre(II).

11. Kit selon l'une des revendications 1 à 10, **caractérisé en ce que** l'au moins un sel d'halogénure est sélectionné parmi le groupe constitué d'halogénures métalliques, d'hydrochlorures et de sels d'halogénure d'ammonium quaternaires.

12. Kit selon l'une des revendications 1 à 11, **caractérisé en ce que** l'au moins un réticulant est sélectionné parmi le groupe constitué de monomères de méthacrylate multifonctionnels.

13. Kit selon la revendication 12, **caractérisé en ce que** le monomère de méthacrylate multifonctionnel est sélectionné parmi le groupe constitué de diméthacrylate d'éthylène-glycol, de diméthacrylate de butylène-glycol et de diméthacrylate d'héxaméthylène.

14. Kit selon l'une des revendications 1 à 13, **caractérisé en ce qu'**au moins une des composantes (a), (b) et (c) du kit contient au moins une substance pharmaceutique active.

15. Kit selon la revendication 14, **caractérisé en ce que** l'au moins une substance pharmaceutique active est sélectionnée parmi le groupe constitué d'antibiotiques, d'antiphlogistiques, d'hormones, de facteurs de croissance, de bisphosphonates et de cytostatiques.

16. Kit selon l'une des revendications 1 à 15, **caractérisé en ce qu'**au moins une des composantes (a), (b) et (c) du kit contient un opacifiant pour radiologie.

17. Kit selon l'une des revendications 1 à 16, **caractérisé en ce qu'**au moins une des composantes (a), (b) et (c) du kit contient un colorant.

18. Pâte comprenant
(i) 15 -50 pourcents en poids d'au moins un ester d'acide méthacrylique hydrophobe monofonctionnel,
(ii) 40 - 85 pourcents en poids d'au moins une charge,
(iii) 0,01 - 4 pourcents en poids d'au moins un initiateur de radical soluble dans l'ester d'acide méthacrylique (i) qui présente un groupe peroxyde,
(iv) 0,01 - 4 pourcents en poids d'au moins un initiateur de radical soluble dans l'ester d'acide méthacrylique (i) qui ne présente pas de groupe peroxyde,
(v) 0,000001 - 3 pourcents en poids d'au moins un accélérateur soluble dans l'ester d'acide méthacrylique (i) qui est capable de former des radicaux à partir des initiateurs de radical selon (iii),
(vi) 0,001 - 5 pourcents en poids d'au moins un sel d'halogénure et
(vii) 0,2 - 3 pourcents en poids d'au moins un réticulant.

19. Emploi d'un kit selon l'une des revendications 1 à 17 ou d'une pâte selon la revendication 18 pour fixer des endoprothèses d'articulation.

20. Emploi d'un kit selon l'une des revendications 1 à 17 ou d'une pâte selon la revendication 18 pour remplir des déficits osseux.
